# EUROPEAN PATENT APPLICATION

(11) **EP 4 714 978 A1**
(43) Date of publication of application: **25.03.2026**
(21) Application number: 24806375.2
(22) Date of filing: 30.04.2024
(51) Int. Cl.: C07K 16/30, C07K 16/28, C07K 19/00, C12N 5/10, A61K 39/00, A61P 35/00

(54) **ANTIBODY TARGETING DLL3 AND USE OF ANTIBODY**

(30) Priority: 17.05.2023 CN 202310557360
(71) Applicant: Neukio Biotherapeutics (Shangai ) Co., Ltd., Shanghai 200131 (CN)
(72) Inventor: LONG, Ting, Shanghai 200131 (CN); ZHANG, Lei, Shanghai 200131 (CN); WANG, Liqun Richard, Shanghai 200131 (CN)
(74) Representative: Mewburn Ellis LLP
(86) International application number: PCT/CN2024/090961
(87) International publication number: WO 2024/235019

(57) **Abstract**

An antibody targeting DLL3 and a use of the antibody. The DLL3 antibody shows excellent specificity and binding capacity, a chimeric antigen receptor derived therefrom shows excellent killing capacity against tumor cells, the cytotoxicity is low, and the side effects are low.

## Description

### Technical field

The present invention relates to the field of antibodies. Specifically, it relates to an antibody targeting DLL3 and a use of the antibody.

### Background

DLL3 protein, full name Delta-like protein 3, is a type I transmembrane protein encoded by the DLL3 gene. It consists of 618 amino acid residues and can be divided into an extracellular Delta/Serrate/Lag-2 (DSL) domain, six EGF-like domains, a transmembrane segment, and an intracellular segment.

During development, DLL3 protein is mainly expressed in the presomitic mesoderm in the early stage of embryonic development. Its mutation leads to Spondylocostal dysostosis in both humans and mice. In adult tissues, DLL3 mRNA can be detected in the brain, endocrine tissues, and testes, but the protein is hardly detectable. This indicates that DLL3 protein has good safety as a target for CAR-NK therapy. At the cellular level, DLL3 protein is mainly localized in the Golgi apparatus rather than the cell membrane surface. When DLL3 is abnormally overexpressed in cells, the DLL3 protein can be detected on the cell membrane surface. In addition, its overexpression is positively correlated with the expression level of ASCL-1 (Achaete-scute homolog 1), a basic helix-loop-helix transcription factor involved in the fate determination of neuroendocrine cells.

DLL3 protein is highly expressed in various solid tumors, including small cell lung cancer, large cell neuroendocrine lung carcinoma, gastroenteropancreatic neuroendocrine carcinoma, castrate resistant neuroendocrine prostate cancer, endometrial cancer, isocitrate dehydrogenase-mutant glioma, and Merkel cell carcinoma, etc. In small cell lung cancer and endometrial cancer, high expression of DLL3 protein is associated with shorter overall survival of patients. Regarding the mechanism of cancer development, as a ligand of the Notch signaling pathway, DLL3 mainly downregulates Notch receptor proteins in DLL3-highly expressed cells through cis-inhibition, thereby inhibiting the Notch signaling pathway. Knockdown of DLL3 in gastrointestinal neuroendocrine cancer cell lines will inhibit cell proliferation.

In summary, DLL3 is a target widely expressed in neuroendocrine cancers with good safety. Targeting DLL3 protein through antibodies, antibody-drug conjugates, CAR-NK, and other means has broad application prospects.

### Summary of the invention

The purpose of the present invention is to provide a DLL3-targeting antibody and its applications.

In the first aspect of the present invention, provided is a DLL3-targeting antibody or an antigen-binding fragment thereof, wherein the antibody or antigen-binding fragment thereof comprises a heavy chain variable region and a light chain variable region, and wherein the heavy chain variable region and light chain variable region comprise complementarity determining regions (CDRs) selected from the group consisting of:
(1) the heavy chain variable region comprises the following complementarity determining regions (CDRs): (Ab.76)
   HCDR1 set forth in SEQ ID NO: 37,
   HCDR2 set forth in SEQ ID NO: 38, and
   HCDR3 set forth in SEQ ID NO: 39;
   and the light chain variable region comprises the following complementarity determining regions (CDRs):
      LCDR1 set forth in SEQ ID NO: 40,
      LCDR2 set forth in SEQ ID NO: 41, and
      LCDR3 set forth in SEQ ID NO: 42; or
(2) the heavy chain variable region comprises the following complementarity determining regions (CDRs): (Ab.55)
   HCDR1 set forth in SEQ ID NO: 7,
   HCDR2 set forth in SEQ ID NO: 8, and
   HCDR3 set forth in SEQ ID NO: 9;
   and the light chain variable region comprises the following complementarity determining regions (CDRs):
      LCDR1 set forth in SEQ ID NO: 10,
      LCDR2 set forth in SEQ ID NO: 11, and
      LCDR3 set forth in SEQ ID NO: 12; or
(3) the heavy chain variable region comprises the following complementarity determining regions (CDRs): (Ab.67)
   HCDR1 set forth in SEQ ID NO: 19,
   HCDR2 set forth in SEQ ID NO: 20, and
   HCDR3 set forth in SEQ ID NO: 21;
   and the light chain variable region comprises the following complementarity determining regions (CDRs):
      LCDR1 set forth in SEQ ID NO: 22,
      LCDR2 set forth in SEQ ID NO: 23, and
      LCDR3 set forth in SEQ ID NO: 24; or
(4) the heavy chain variable region comprises the following complementarity determining regions (CDRs): (Ab.73)
   HCDR1 set forth in SEQ ID NO: 31,
   HCDR2 set forth in SEQ ID NO: 32, and
   HCDR3 set forth in SEQ ID NO: 33;
   and the light chain variable region comprises the following complementarity determining regions (CDRs):
      LCDR1 set forth in SEQ ID NO: 34,
      LCDR2 set forth in SEQ ID NO: 35, and
      LCDR3 set forth in SEQ ID NO: 36; or
(5) the heavy chain variable region comprises the following complementarity determining regions (CDRs): (Ab.71)
   HCDR1 set forth in SEQ ID NO: 25,
   HCDR2 set forth in SEQ ID NO: 26, and
   HCDR3 set forth in SEQ ID NO: 27;
   and the light chain variable region comprises the following complementarity determining regions (CDRs):
      LCDR1 set forth in SEQ ID NO: 28,
      LCDR2 set forth in SEQ ID NO: 29, and
      LCDR3 set forth in SEQ ID NO: 30; or
(6) the heavy chain variable region comprises the following complementarity determining regions (CDRs): (Ab.53)
   HCDR1 set forth in SEQ ID NO: 1,
   HCDR2 set forth in SEQ ID NO: 2, and
   HCDR3 set forth in SEQ ID NO: 3;
   and the light chain variable region comprises the following complementarity determining regions (CDRs):
      LCDR1 set forth in SEQ ID NO: 4,
      LCDR2 set forth in SEQ ID NO: 5, and
      LCDR3 set forth in SEQ ID NO: 6; or
(7) the heavy chain variable region comprises the following complementarity determining regions (CDRs): (Ab.79)
   HCDR1 set forth in SEQ ID NO: 13,
   HCDR2 set forth in SEQ ID NO: 43, and
   HCDR3 set forth in SEQ ID NO: 33;
   and the light chain variable region comprises the following complementarity determining regions (CDRs):
      LCDR1 set forth in SEQ ID NO: 44,
      LCDR2 set forth in SEQ ID NO: 35, and
      LCDR3 set forth in SEQ ID NO: 36; or
(8) the heavy chain variable region comprises the following complementarity determining regions (CDRs): (Ab.63)
   HCDR1 set forth in SEQ ID NO: 13,
   HCDR2 set forth in SEQ ID NO: 14, and
   HCDR3 set forth in SEQ ID NO: 15;
   and the light chain variable region comprises the following complementarity determining regions (CDRs):
      LCDR1 set forth in SEQ ID NO: 16,
      LCDR2 set forth in SEQ ID NO: 17, and
      LCDR3 set forth in SEQ ID NO: 18;
      wherein the CDR sequences are identified based on the Kabat numbering scheme.

In another preferred embodiment, the heavy chain variable region and light chain variable region of the antibody or antigen-binding fragment thereof comprise complementarity determining regions (CDRs) selected from the group consisting of:
(1) the heavy chain variable region comprises the following complementarity determining regions (CDRs): (Ab.76)
   HCDR1 set forth in SEQ ID NO: 56,
   HCDR2 set forth in SEQ ID NO: 57, and
   HCDR3 set forth in SEQ ID NO: 39;
   and the light chain variable region comprises the following complementarity determining regions (CDRs):
      LCDR1 set forth in SEQ ID NO: 40,
      LCDR2 set forth in SEQ ID NO: 41, and
      LCDR3 set forth in SEQ ID NO: 42; or
(2) the heavy chain variable region comprises the following complementarity determining regions (CDRs): (Ab.55)
   HCDR1 set forth in SEQ ID NO: 47,
   HCDR2 set forth in SEQ ID NO: 48, and
   HCDR3 set forth in SEQ ID NO: 9;
   and the light chain variable region comprises the following complementarity determining regions (CDRs):
      LCDR1 set forth in SEQ ID NO: 10,
      LCDR2 set forth in SEQ ID NO: 11, and
      LCDR3 set forth in SEQ ID NO: 12; or
(3) the heavy chain variable region comprises the following complementarity determining regions (CDRs): (Ab.67)
   HCDR1 set forth in SEQ ID NO: 50,
   HCDR2 set forth in SEQ ID NO: 51, and
   HCDR3 set forth in SEQ ID NO: 21;
   and the light chain variable region comprises the following complementarity determining regions (CDRs):
      LCDR1 set forth in SEQ ID NO: 22,
      LCDR2 set forth in SEQ ID NO: 23, and
      LCDR3 set forth in SEQ ID NO: 24; or
(4) the heavy chain variable region comprises the following complementarity determining regions (CDRs): (Ab.73)
   HCDR1 set forth in SEQ ID NO: 54,
   HCDR2 set forth in SEQ ID NO: 55, and
   HCDR3 set forth in SEQ ID NO: 33;
   and the light chain variable region comprises the following complementarity determining regions (CDRs):
      LCDR1 set forth in SEQ ID NO: 34,
      LCDR2 set forth in SEQ ID NO: 35, and
      LCDR3 set forth in SEQ ID NO: 36; or
(5) the heavy chain variable region comprises the following complementarity determining regions (CDRs): (Ab.71)
   HCDR1 set forth in SEQ ID NO: 52,
   HCDR2 set forth in SEQ ID NO: 53, and
   HCDR3 set forth in SEQ ID NO: 27;
   and the light chain variable region comprises the following complementarity determining regions (CDRs):
      LCDR1 set forth in SEQ ID NO: 28,
      LCDR2 set forth in SEQ ID NO: 29, and
      LCDR3 set forth in SEQ ID NO: 30; or
(6) the heavy chain variable region comprises the following complementarity determining regions (CDRs): (Ab.53)
   HCDR1 set forth in SEQ ID NO: 45,
   HCDR2 set forth in SEQ ID NO: 46, and
   HCDR3 set forth in SEQ ID NO: 3;
   and the light chain variable region comprises the following complementarity determining regions (CDRs):
      LCDR1 set forth in SEQ ID NO: 4,
      LCDR2 set forth in SEQ ID NO: 5, and
      LCDR3 set forth in SEQ ID NO: 6; or
(7) the heavy chain variable region comprises the following complementarity determining regions (CDRs): (Ab.79)
   HCDR1 set forth in SEQ ID NO: 58,
   HCDR2 set forth in SEQ ID NO: 48, and
   HCDR3 set forth in SEQ ID NO: 33;
   and the light chain variable region comprises the following complementarity determining regions (CDRs):
      LCDR1 set forth in SEQ ID NO: 44,
      LCDR2 set forth in SEQ ID NO: 35, and
      LCDR3 set forth in SEQ ID NO: 36; or
(8) the heavy chain variable region comprises the following complementarity determining regions (CDRs): (Ab.63)
   HCDR1 set forth in SEQ ID NO: 49,
   HCDR2 set forth in SEQ ID NO: 48, and
   HCDR3 set forth in SEQ ID NO: 15;
   and the light chain variable region comprises the following complementarity determining regions (CDRs):
      LCDR1 set forth in SEQ ID NO: 16,
      LCDR2 set forth in SEQ ID NO: 17, and
      LCDR3 set forth in SEQ ID NO: 18;
      wherein the CDR sequences are identified based on the Chothia numbering scheme.

In another preferred embodiment, the antibody comprises a heavy chain and a light chain, wherein the heavy chain comprises the three aforementioned heavy chain CDRs and heavy chain framework regions for connecting the heavy chain CDRs; and wherein the light chain comprises the three aforementioned light chain CDRs and light chain framework regions for connecting the light chain CDRs.

In another preferred embodiment, the DLL3-targeting antibody or antigen-binding fragment thereof is selected from the group consisting of: camelid Ig, Ig NAR, Fab fragment, Fab' fragment, F(ab)'2 fragment, F(ab)'3 fragment, Fv, single-chain Fv antibody ("scFv"), bispecific scFv, (scFv)2, minibody, diabody, triabody, tetrabody, disulfide-stabilized Fv protein ("dsFv"), and single-domain antibody (sdAb, nanobody).

In another preferred embodiment, the light chain of the antibody further comprises a light chain constant region.

In another preferred embodiment, the light chain constant region is human-derived, murine-derived, or rabbit-derived, preferably human-derived.

In another preferred embodiment, the heavy chain of the antibody further comprises a heavy chain constant region.

In another preferred embodiment, the heavy chain constant region is human-derived, murine-derived, or rabbit-derived, preferably human-derived.

In another preferred embodiment, the antibody is a double-chain antibody or a single-chain antibody.

In another preferred embodiment, the antibody is a monoclonal antibody.

In another preferred embodiment, the antibody comprises a monospecific, bispecific, trispecific, or multispecific antibody.

In another preferred embodiment, the antibody specifically binds to DLL3.

In another preferred embodiment, the KD value (M) of the antibody for the affinity to human DLL3 is 1.0E-12 to 1.0E-8.

In another preferred embodiment, the antibody is a single-chain antibody (scFv) having an amino acid sequence selected from the group consisting of: SEQ ID NOs: 59, 60, 61, 62, 63, 64, 65, and 66.

In another preferred embodiment, the single-chain antibody has at least 80%, 85%, 90%, 91%, 92%, 93%, 94%, 95%, 96%, 97%, 98%, or 99% sequence homology or sequence identity to the amino acid sequence set forth in SEQ ID NOs: 59, 60, 61, 62, 63, 64, 65, or 66 (wherein the CDRs are unchanged or substantially unchanged).

In the second aspect of the present invention, provided is a recombinant protein, wherein the recombinant protein comprises:
(i) the antibody or antigen-binding fragment thereof according to the first aspect of the present invention; and
(ii) an optional tag sequence facilitating expression and/or purification.

In another preferred embodiment, the tag sequence comprises a 6His tag.

In another preferred embodiment, the recombinant protein (or polypeptide) comprises a fusion protein.

In another preferred embodiment, the recombinant protein is a monomer, dimer, or multimer.

In another preferred embodiment, the recombinant protein further comprises an additional fusion element (or fusion polypeptide fragment) fused with the element (i).

In the third aspect of the present invention, provided is a chimeric antigen receptor (CAR), wherein the antigen-binding domain of the chimeric antigen receptor contains an antibody single-chain variable region sequence (scFv) targeting DLL3, and wherein the heavy chain variable region and light chain variable region of the scFv comprise complementarity determining regions (CDRs) selected from the group consisting of:
(1) the heavy chain variable region comprises the following complementarity determining regions (CDRs): (Ab.76)
   HCDR1 set forth in SEQ ID NO: 37,
   HCDR2 set forth in SEQ ID NO: 38, and
   HCDR3 set forth in SEQ ID NO: 39;
   and the light chain variable region comprises the following complementarity determining regions (CDRs):
      LCDR1 set forth in SEQ ID NO: 40,
      LCDR2 set forth in SEQ ID NO: 41, and
      LCDR3 set forth in SEQ ID NO: 42; or
(2) the heavy chain variable region comprises the following complementarity determining regions (CDRs): (Ab.55)
   HCDR1 set forth in SEQ ID NO: 7,
   HCDR2 set forth in SEQ ID NO: 8, and
   HCDR3 set forth in SEQ ID NO: 9;
   and the light chain variable region comprises the following complementarity determining regions (CDRs):
      LCDR1 set forth in SEQ ID NO: 10,
      LCDR2 set forth in SEQ ID NO: 11, and
      LCDR3 set forth in SEQ ID NO: 12; or
(3) the heavy chain variable region comprises the following complementarity determining regions (CDRs): (Ab.67)
   HCDR1 set forth in SEQ ID NO: 19,
   HCDR2 set forth in SEQ ID NO: 20, and
   HCDR3 set forth in SEQ ID NO: 21;
   and the light chain variable region comprises the following complementarity determining regions (CDRs):
      LCDR1 set forth in SEQ ID NO: 22,
      LCDR2 set forth in SEQ ID NO: 23, and
      LCDR3 set forth in SEQ ID NO: 24; or
(4) the heavy chain variable region comprises the following complementarity determining regions (CDRs): (Ab.73)
   HCDR1 set forth in SEQ ID NO: 31,
   HCDR2 set forth in SEQ ID NO: 32, and
   HCDR3 set forth in SEQ ID NO: 33;
   and the light chain variable region comprises the following complementarity determining regions (CDRs):
      LCDR1 set forth in SEQ ID NO: 34,
      LCDR2 set forth in SEQ ID NO: 35, and
      LCDR3 set forth in SEQ ID NO: 36; or
(5) the heavy chain variable region comprises the following complementarity determining regions (CDRs): (Ab.71)
   HCDR1 set forth in SEQ ID NO: 25,
   HCDR2 set forth in SEQ ID NO: 26, and
   HCDR3 set forth in SEQ ID NO: 27;
   and the light chain variable region comprises the following complementarity determining regions (CDRs):
      LCDR1 set forth in SEQ ID NO: 28,
      LCDR2 set forth in SEQ ID NO: 29, and
      LCDR3 set forth in SEQ ID NO: 30; or
(6) the heavy chain variable region comprises the following complementarity determining regions (CDRs): (Ab.53)
   HCDR1 set forth in SEQ ID NO: 1,
   HCDR2 set forth in SEQ ID NO: 2, and
   HCDR3 set forth in SEQ ID NO: 3;
   and the light chain variable region comprises the following complementarity determining regions (CDRs):
      LCDR1 set forth in SEQ ID NO: 4,
      LCDR2 set forth in SEQ ID NO: 5, and
      LCDR3 set forth in SEQ ID NO: 6; or
(7) the heavy chain variable region comprises the following complementarity determining regions (CDRs): (Ab.79)
   HCDR1 set forth in SEQ ID NO: 13,
   HCDR2 set forth in SEQ ID NO: 43, and
   HCDR3 set forth in SEQ ID NO: 33;
   and the light chain variable region comprises the following complementarity determining regions (CDRs):
      LCDR1 set forth in SEQ ID NO: 44,
      LCDR2 set forth in SEQ ID NO: 35, and
      LCDR3 set forth in SEQ ID NO: 36; or
(8) the heavy chain variable region comprises the following complementarity determining regions (CDRs): (Ab.63)
   HCDR1 set forth in SEQ ID NO: 13,
   HCDR2 set forth in SEQ ID NO: 14, and
   HCDR3 set forth in SEQ ID NO: 15;
   and the light chain variable region comprises the following complementarity determining regions (CDRs):
      LCDR1 set forth in SEQ ID NO: 16,
      LCDR2 set forth in SEQ ID NO: 17, and
      LCDR3 set forth in SEQ ID NO: 18;
      wherein the CDR sequences are identified based on the Kabat numbering scheme.

In another preferred embodiment, the heavy chain variable region and light chain variable region of the scFv comprise complementarity determining regions (CDRs) selected from the group consisting of:
(1) the heavy chain variable region comprises the following complementarity determining regions (CDRs): (Ab.76)
   HCDR1 set forth in SEQ ID NO: 56,
   HCDR2 set forth in SEQ ID NO: 57, and
   HCDR3 set forth in SEQ ID NO: 39;
   and the light chain variable region comprises the following complementarity determining regions (CDRs):
      LCDR1 set forth in SEQ ID NO: 40,
      LCDR2 set forth in SEQ ID NO: 41, and
      LCDR3 set forth in SEQ ID NO: 42; or
(2) the heavy chain variable region comprises the following complementarity determining regions (CDRs): (Ab.55)
   HCDR1 set forth in SEQ ID NO: 47,
   HCDR2 set forth in SEQ ID NO: 48, and
   HCDR3 set forth in SEQ ID NO: 9;
   and the light chain variable region comprises the following complementarity determining regions (CDRs):
      LCDR1 set forth in SEQ ID NO: 10,
      LCDR2 set forth in SEQ ID NO: 11, and
      LCDR3 set forth in SEQ ID NO: 12; or
(3) the heavy chain variable region comprises the following complementarity determining regions (CDRs): (Ab.67)
   HCDR1 set forth in SEQ ID NO: 50,
   HCDR2 set forth in SEQ ID NO: 51, and
   HCDR3 set forth in SEQ ID NO: 21;
   and the light chain variable region comprises the following complementarity determining regions (CDRs):
      LCDR1 set forth in SEQ ID NO: 22,
      LCDR2 set forth in SEQ ID NO: 23, and
      LCDR3 set forth in SEQ ID NO: 24; or
(4) the heavy chain variable region comprises the following complementarity determining regions (CDRs): (Ab.73)
   HCDR1 set forth in SEQ ID NO: 54,
   HCDR2 set forth in SEQ ID NO: 55, and
   HCDR3 set forth in SEQ ID NO: 33;
   and the light chain variable region comprises the following complementarity determining regions (CDRs):
      LCDR1 set forth in SEQ ID NO: 34,
      LCDR2 set forth in SEQ ID NO: 35, and
      LCDR3 set forth in SEQ ID NO: 36; or
(5) the heavy chain variable region comprises the following complementarity determining regions (CDRs): (Ab.71)
   HCDR1 set forth in SEQ ID NO: 52,
   HCDR2 set forth in SEQ ID NO: 53, and
   HCDR3 set forth in SEQ ID NO: 27;
   and the light chain variable region comprises the following complementarity determining regions (CDRs):
      LCDR1 set forth in SEQ ID NO: 28,
      LCDR2 set forth in SEQ ID NO: 29, and
      LCDR3 set forth in SEQ ID NO: 30; or
(6) the heavy chain variable region comprises the following complementarity determining regions (CDRs): (Ab.53)
   HCDR1 set forth in SEQ ID NO: 45,
   HCDR2 set forth in SEQ ID NO: 46, and
   HCDR3 set forth in SEQ ID NO: 3;
   and the light chain variable region comprises the following complementarity determining regions (CDRs):
      LCDR1 set forth in SEQ ID NO: 4,
      LCDR2 set forth in SEQ ID NO: 5, and
      LCDR3 set forth in SEQ ID NO: 6; or
(7) the heavy chain variable region comprises the following complementarity determining regions (CDRs): (Ab.79)
   HCDR1 set forth in SEQ ID NO: 58,
   HCDR2 set forth in SEQ ID NO: 48, and
   HCDR3 set forth in SEQ ID NO: 33;
   and the light chain variable region comprises the following complementarity determining regions (CDRs):
      LCDR1 set forth in SEQ ID NO: 44,
      LCDR2 set forth in SEQ ID NO: 35, and
      LCDR3 set forth in SEQ ID NO: 36; or
(8) the heavy chain variable region comprises the following complementarity determining regions (CDRs): (Ab.63)
   HCDR1 set forth in SEQ ID NO: 49,
   HCDR2 set forth in SEQ ID NO: 48, and
   HCDR3 set forth in SEQ ID NO: 15;
   and the light chain variable region comprises the following complementarity determining regions (CDRs):
      LCDR1 set forth in SEQ ID NO: 16,
      LCDR2 set forth in SEQ ID NO: 17, and
      LCDR3 set forth in SEQ ID NO: 18;
      wherein the CDR sequences are identified based on the Chothia numbering scheme.

In another preferred embodiment, the scFv further comprises a linker peptide between the heavy chain variable region and the light chain variable region.

In another preferred embodiment, the linker peptide is (G4S)3 or (G4S)4.

In another preferred embodiment, the scFv is represented by Formula A or Formula B as follows:

VH-VL, (A);

VL-VH, (B)

wherein, VH is the heavy chain variable region of the antibody; VL is the light chain variable region of the antibody; and "-" is a linker peptide or a peptide bond.

In another preferred embodiment, the linker peptide is (G4S)n, wherein n is preferably 3-5; and more preferably n is 3.

In another preferred embodiment, the scFv has an amino acid sequence selected from the group consisting of: SEQ ID NOs: 59, 60, 61, 62, 63, 64, 65, and 66.

In another preferred embodiment, the antibody single-chain variable region comprises human-derived, murine-derived, or human-murine chimeric antibody single-chain variable regions.

In another preferred embodiment, the scFv is represented by Formula A (VH-VL).

In another preferred embodiment, the antigen-binding domain targets the extracellular region of DLL3.

In another preferred embodiment, the chimeric antigen receptor has a structure of Formula I as follows:

L-scFv-H-TM-C-CD3ζ (I)

wherein,
each "-" is independently a linker peptide or a peptide bond;
L is absent or a signal peptide sequence;
scFv is the scFv targeting DLL3;
H is an optional hinge region;
TM is a transmembrane domain;
C is a costimulatory signaling molecule;
CD3ζ is the CD3ζ cytoplasmic signaling sequence.

In another preferred embodiment, L is a signal peptide of a protein selected from the group consisting of: CD8, CD4, CD16, CD56, CD137, CSF2, DAP12, EF1, GM-CSF, IL-8, IL-21, and a combination thereof.

In another preferred embodiment, L is a CD8-derived signal peptide.

In another preferred embodiment, the scFv is represented by Formula A or Formula B as follows:

VH-VL, (A);

VL-VH, (B)

wherein, VH is the heavy chain variable region of the antibody; VL is the light chain variable region of the antibody; and "-" is a linker peptide or a peptide bond.

In another preferred embodiment, the scFv is represented by Formula A (VH-VL).

In another preferred embodiment, H is a hinge region of a protein selected from the group consisting of: CD8, CD28, CD137, Fc, and a combination thereof.

In another preferred embodiment, H is a CD28-derived hinge region.

In another preferred embodiment, TM is a transmembrane region of a protein selected from the group consisting of: CD28, CD3 epsilon, CD45, CD4, CD5, CD8, CD9, CD16, CD22, CD33, CD37, CD64, CD80, CD86, CD134, CD137, CD154, and a combination thereof.

In another preferred embodiment, TM is a CD28-derived transmembrane region.

In another preferred embodiment, C is a costimulatory signaling molecule of a protein selected from the group consisting of: OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, TLR2, and a combination thereof.

In another preferred embodiment, C is a CD28-derived costimulatory signaling molecule.

In the fourth aspect of the present invention, provided is a polynucleotide, wherein the polynucleotide encodes the antibody or antigen-binding fragment thereof according to the first aspect of the present invention, the recombinant protein according to the second aspect of the present invention, or the chimeric antigen receptor (CAR) according to the third aspect of the present invention.

In another preferred embodiment, the polynucleotide is isolated.

In the fifth aspect of the present invention, provided is a vector, wherein the vector comprises the polynucleotide according to the fourth aspect of the present invention.

In another preferred embodiment, the vector is selected from the group consisting of: DNA, RNA, plasmid, lentiviral vector, adenoviral vector, retroviral vector, transposon, and a combination thereof.

In another preferred embodiment, the vector is a retroviral vector.

In the sixth aspect of the present invention, provided is a host cell, wherein the host cell comprises the vector according to the fifth aspect of the present invention, or has an exogenous polynucleotide according to the fourth aspect of the present invention integrated into its chromosome.

In another preferred embodiment, the cell is an isolated cell, and/or the cell is a genetically engineered cell.

In another preferred embodiment, the cell is a mammalian cell.

In another preferred embodiment, the cell is an NK cell or a T cell.

In another preferred embodiment, the host cell is an engineered immune cell.

In another preferred embodiment, the engineered immune cell comprises a T cell or an NK cell, preferably being (i) a chimeric antigen receptor T cell (CAR-T cell); or (ii) a chimeric antigen receptor NK cell (CAR-NK cell), wherein the sources of NK cells include peripheral blood, umbilical cord blood, embryonic stem cells (ESCs), induced pluripotent stem cells (iPSCs), and the like.

In another preferred embodiment, the host cell is an immune cell, and the immune cell expresses or exposes on the cell membrane the antibody according to the first aspect of the present invention or the chimeric antigen receptor according to the third aspect of the present invention.

In another preferred embodiment, the immune cell comprises an NK cell or a T cell.

In another preferred embodiment, the immune cell is derived from a human or a non-human mammal (e.g., a mouse).

In the seventh aspect of the present invention, provided is a method for preparing CAR-NK cells or CAR-T cells, wherein the CAR-NK cells or CAR-T cells express the chimeric antigen receptor according to the third aspect of the present invention, comprising the following steps:
transducing the polynucleotide according to the fourth aspect of the present invention or the vector according to the fifth aspect of the present invention into NK cells or T cells, thereby obtaining the CAR-NK cells or CAR-T cells.

In the eighth aspect of the present invention, provided is a pharmaceutical composition, wherein the pharmaceutical composition comprises the antibody or antigen-binding fragment thereof according to the first aspect of the present invention, the recombinant protein according to the second aspect of the present invention, the chimeric antigen receptor according to the third aspect of the present invention, the polynucleotide according to the fourth aspect of the present invention, the vector according to the fifth aspect of the present invention, or the host cell according to the sixth aspect of the present invention, and a pharmaceutically acceptable carrier, diluent or excipient.

In another preferred embodiment, the pharmaceutical composition is a preparation, preferably a liquid preparation.

In another preferred embodiment, the dosage form of the pharmaceutical composition is an injection.

In another preferred embodiment, the pharmaceutical composition is used for the manufacture of a medicament or preparation for preventing and/or treating cancer or tumor.

In the ninth aspect of the present invention, provided is an immunoconjugate, wherein the immunoconjugate comprises:
(a) an antibody moiety selected from the group consisting of: the antibody or antigen-binding fragment thereof according to the first aspect of the present invention, the recombinant protein according to the second aspect of the present invention, and a combination thereof; and
(b) a conjugated moiety coupled to the antibody moiety, wherein the conjugated moiety is selected from the group consisting of: a detectable label, a drug, a toxin, a cytokine, a radionuclide, an enzyme, and a combination thereof.

In another preferred embodiment, the conjugated moiety is selected from: a fluorescent or luminescent label, a radioactive label, an MRI (magnetic resonance imaging) or CT (computed tomography) contrast agent, an enzyme capable of producing a detectable product, a radionuclide, a biological toxin, a cytokine (e.g., IL-2, etc.), an antibody, an antibody Fc fragment, an antibody scFv fragment, gold nanoparticles/nanorods, viral particles, liposomes, magnetic nanoparticles, a prodrug-activating enzyme (e.g., DT-diaphorase (DTD) or biphenyl hydrolase-like protein (BPHL)), a chemotherapeutic agent (e.g., cisplatin), or nanoparticles of any form, and the like.

In the tenth aspect of the present invention, provided is a use of the antibody or antigen-binding fragment thereof according to the first aspect of the present invention, the recombinant protein according to the second aspect of the present invention, the chimeric antigen receptor according to the third aspect of the present invention, the polynucleotide according to the fourth aspect of the present invention, the vector according to the fifth aspect of the present invention, the host cell according to the sixth aspect of the present invention, the pharmaceutical composition according to the eighth aspect of the present invention, or the immunoconjugate according to the ninth aspect of the present invention, for:
(a) manufacturing a detection reagent or kit; and/or
(b) manufacturing a medicament or preparation for preventing and/or treating DLL3-associated diseases.

In another preferred embodiment, the DLL3-associated disease is cancer or tumor.

In another preferred embodiment, the tumor is selected from the group consisting of: hematological tumor, solid tumor, and a combination thereof.

In another preferred embodiment, the solid tumor is selected from the group consisting of: breast cancer, gastric cancer, lung cancer, ovarian cancer, colorectal cancer, pancreatic cancer, endometrial cancer, melanoma, mesothelioma, and a combination thereof.

In another preferred embodiment, the tumor is a DLL3-positive tumor; preferably selected from the group consisting of: chronic lymphoma, breast cancer, lung cancer, ovarian cancer, colorectal cancer, endometrial cancer, melanoma, small cell lung cancer (SCLC), large cell neuroendocrine lung carcinoma (LCNEC), gastroenteropancreatic neuroendocrine carcinoma, castrate resistant neuroendocrine prostate cancers (crNEPC), small-cell bladder cancer, neuroendocrine carcinoma of the uterine cervix, isocitrate dehydrogenase-mutant glioma, Merkel cell carcinoma, medullary thyroid carcinomas, and a combination thereof.

In another preferred embodiment, the detection is an immunoassay.

In another preferred embodiment, the immunoassay is an ELISA immunoassay, immunochromatographic assay, immunocytochemistry staining assay, or immunohistochemistry staining assay.

In another preferred embodiment, the diagnostic reagent is a detection strip or a detection plate.

In another preferred embodiment, the reagent comprises a chip, or antibody-coated immunomicroparticles.

In the eleventh aspect of the present invention, provided is a method for *in vitro* detection (including diagnostic or non-diagnostic) of DLL3 protein in a sample, comprising the steps of:
(1) contacting the sample with the antibody or antigen-binding fragment thereof according to the first aspect of the present invention, or the recombinant protein according to the second aspect of the present invention;
(2) detecting whether an antigen-antibody complex is formed, wherein the formation of the complex indicates the presence of DLL3 protein in the sample.

In another preferred embodiment, the diagnostic reagent is a detection strip or a detection plate.

In another preferred embodiment, the method is an immunocytochemistry staining (ICC) assay, an immunohistochemistry (IHC) assay, a whole cell ELISA assay, or a cell lysate ELISA assay.

In the twelfth aspect of the present invention, provided is a method for preparing a recombinant polypeptide, wherein the method comprises:
(a) culturing the host cell according to the sixth aspect of the present invention under conditions suitable for expression;
(b) isolating the recombinant polypeptide from the culture, wherein the recombinant polypeptide is the antibody or antigen-binding fragment thereof according to the first aspect of the present invention, or the recombinant protein according to the second aspect of the present invention.

In the thirteenth aspect of the present invention, provided is a detection plate, comprising: a substrate (support plate) and a test strip, wherein the test strip contains the antibody or antigen-binding fragment thereof according to the first aspect of the present invention, the recombinant protein according to the second aspect of the present invention, the immunoconjugate according to the ninth aspect of the present invention, or a combination thereof.

In the fourteenth aspect of the present invention, provided is a kit, comprising:
(1) a first container containing the antibody or antigen-binding fragment thereof according to the first aspect of the present invention; and/or
(2) a second container containing a secondary antibody against the antibody of the present invention;
or, the kit comprises the detection plate according to the thirteenth aspect of the present invention.

In the fifteenth aspect of the present invention, provided is a method for treating a disease, comprising administering an appropriate amount of the antibody or antigen-binding fragment thereof according to the first aspect of the present invention, the recombinant protein according to the second aspect of the present invention, the host cell according to the twelfth aspect of the present invention, or the pharmaceutical composition according to the eighth aspect of the present invention, to a subject in need thereof.

In another preferred embodiment, the disease is cancer or tumor.

It should be understood that within the scope of the present invention, the above-mentioned technical features of the present invention and the technical features specifically described in the following (such as the examples) can be combined with each other to form a new or preferred technical solution, which is not redundantly repeated one by one herein due to space limitation.

### Description of the drawings

Figure 1 shows the SDS-PAGE results of the anti-DLL3 scFv-Fc antibodies.
Figure 2 shows the BLI assay results of the anti-DLL3 antibodies.
Figure 3 shows the EC50 assay results of the anti-DLL3 antibodies.
Figure 4 shows the CAR molecular structure of the anti-DLL3 antibody.
Figure 5 shows the detection results of DLL3 protein expression on the surface of tumor cell lines.
Figure 6 shows the CAR detection results of the sorted anti-DLL3 CAR-NK92 cell lines.
Figure 7 shows the single-round killing results of anti-DLL3 CAR-NK92 cell lines against SHP77 and NCI-H82 cells.
Figure 8A shows the multi-round killing results of anti-DLL3 CAR-NK92 cell lines against SHP77 cells, and Figure 8B shows the multi-round killing results of anti-DLL3 CAR-NK92 cell lines against NCI-H82 cells.

### Detailed description

After extensive and in-depth research, the inventors have unexpectedly obtained a series of DLL3-targeting antibodies for the first time. The screened antibodies have excellent biological activity. Further, based on these antibodies, a chimeric antigen receptor structure targeting DLL3 was constructed. NK cells expressing the chimeric antigen receptor exhibit superior killing ability against target cells. On this basis, the present invention has been completed.

### Terms

Unless otherwise defined, all technical and scientific terms used herein have the same meaning as commonly understood by those of ordinary skill in the art to which the present invention belongs.

The term "about" may refer to a value or composition within an acceptable error range for a particular value or composition as determined by one of ordinary skill in the art, which will depend in part on how the value or composition is measured or determined.

As used herein, the term "containing" or "including (comprising)" may be open, semi-closed, or closed. In other words, the term also includes "consisting essentially of" or "consisting of".

### Antibody

The term "antibody" (Ab) shall include, but not be limited to, immunoglobulins that specifically bind to an antigen and comprise at least two heavy (H) chains and two light (L) chains interconnected by disulfide bonds, or antigen-binding portions thereof. Each H chain comprises a heavy chain variable region (abbreviated herein as VH) and a heavy chain constant region. The heavy chain constant region comprises three constant domains CH1, CH2, and CH3. Each L chain comprises a light chain variable region (abbreviated herein as VL) and a light chain constant region. The light chain constant region comprises one constant domain CL. The VH and VL regions can be further subdivided into regions of hypervariability called complementarity determining regions (CDRs), interspersed with more conserved regions called framework regions (FRs). Each of VH and VL comprises three CDRs and four FRs, arranged from the amino-terminus to the carboxy-terminus in the following order: FR1, CDR1, FR2, CDR2, FR3, CDR3, FR4. The variable regions of the heavy and light chains contain the binding domains that interact with the antigen.

As used herein, the terms "heavy chain variable region" and "VH" may be used interchangeably.

As used herein, the terms "light chain variable region" and "VL" may be used interchangeably.

As used herein, terms such as "antigen-binding domain" include any naturally occurring, enzymatically obtainable, synthetic, or genetically engineered polypeptide or glycoprotein that specifically binds to an antigen to form a complex. Antigen-binding fragments of antibodies can be derived from intact antibody molecules using any suitable standard technique such as proteolytic digestion or recombinant genetic engineering techniques involving manipulating and expressing DNA encoding antibody variable domains and optionally antibody constant domains. Such DNA is known and/or readily available from, for example, commercial sources, DNA libraries (including, for example, phage antibody libraries), or can be synthesized. The DNA can be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments or antigen-binding domains as used herein include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of amino acid residues that mimic the hypervariable regions of antibodies (e.g., isolated complementarity determining regions (CDRs) such as CDR3 peptides) or constrained FR3-CDR3-FR4 peptides.

As used herein, an antigen-binding fragment or antigen-binding domain will generally comprise at least one variable domain. The variable domain may be of any size or amino acid composition and will typically comprise at least one CDR adjacent to or in reading frame with one or more framework sequences. In antigen-binding fragments having a VH domain associated with a VL domain, the VH and VL domains may be arranged relative to each other in any suitable configuration. For example, the variable region may be dimeric and contain VH-VH, VH-VL, or VL-VL dimers. Alternatively, the antigen-binding domain may contain a monomeric VH or VL domain.

In the amino acid sequence of the light chain variable region or heavy chain variable region of a given antibody, the precise amino acid sequence boundaries of each CDR can be determined using any one or combination of many well-known antibody CDR assignment systems, including, for example: Chothia based on the three-dimensional structure of antibodies and the topology of CDR loops, Kabat based on antibody sequence variability (Kabat, E., et al., U.S. Department of Health and Human Services, Sequences of Proteins of Immunological Interest, (1983)), AbM (University of Bath), Contact (University College London), International Immuno GeneTics database (IMGT), EU numbering system, and Chothia definition based on loop structure positions.

It should be understood that the precise amino acid sequence boundaries of the CDRs in the present invention may optionally be defined using the different assignment systems mentioned above. Preferably, unless otherwise stated, in the present invention, when referring to residue positions in antibody variable regions (including heavy chain variable region residues and light chain variable region residues), it refers to the numbered positions according to the Kabat numbering system.

As used herein, the term "antibody" or "immunoglobulin" is a heterotetrameric glycoprotein of approximately 150,000 Daltons with the same structural characteristics, consisting of two identical light chains (L) and two identical heavy chains (H). Each light chain is linked to a heavy chain by a covalent disulfide bond, and the number of disulfide bonds between heavy chains varies among different immunoglobulin isotypes. Each heavy and light chain also has regularly spaced intrachain disulfide bonds. One end of each heavy chain has a variable region (VH), followed by a plurality of constant regions. One end of each light chain has a variable region (VL), and the other end has a constant region; the constant region of the light chain is opposite the first constant region of the heavy chain, and the variable region of the light chain is opposite the variable region of the heavy chain. Special amino acid residues form an interface between the variable regions of the light and heavy chains.

As used herein, the term "variable" indicates that certain parts of the variable regions in antibodies differ in sequence, which confers the binding specificity of each particular antibody for its particular antigen. However, variability is not uniformly distributed throughout the antibody variable regions. It is concentrated in three fragments called complementarity determining regions (CDRs) or hypervariable regions in the variable regions of light and heavy chains. The more conserved parts of the variable regions are called framework regions (FRs). The variable regions of natural heavy and light chains each contain four FR regions, which are roughly in a β-sheet configuration, connected by three CDRs forming connecting loops, and in some cases can form part of the β-sheet structure. The CDRs in each chain are closely adjacent to each other through the FR regions and together with the CDRs of the other chain form the antigen-binding site of the antibody (see Kabat et al., NIH Publ. No. 91-3242, Volume I, pp. 647-669 (1991)). The constant regions are not directly involved in the binding of the antibody to the antigen, but they exhibit different effector functions, such as participating in antibody-dependent cellular cytotoxicity.

The present invention includes not only intact monoclonal antibodies but also fragments of antibodies with immunological activity or fusion proteins formed by antibodies with other sequences. Therefore, the present invention also includes fragments, derivatives, and analogs of the antibodies.

As used herein, the terms "fragment", "derivative", and "analog" refer to polypeptides that substantially retain the same biological function or activity as the antibody of the present invention. The polypeptide fragments, derivatives, or analogs of the present invention may be (i) polypeptides with one or more conservative or non-conservative amino acid residues (preferably conservative amino acid residues) substituted, and such substituted amino acid residues may or may not be encoded by the genetic code, or (ii) polypeptides with substituted groups in one or more amino acid residues, or (iii) polypeptides formed by fusing a mature polypeptide with another compound (such as a compound that extends the half-life of the polypeptide, such as polyethylene glycol), or (iv) polypeptides formed by fusing additional amino acid sequences to this polypeptide sequence (such as a leader sequence or secretory sequence or a sequence used to purify this polypeptide or a proprotein sequence, or a fusion protein formed with a 6His tag). Based on the teachings herein, these fragments, derivatives, and analogs are within the scope well known to those skilled in the art.

The "light chains" of vertebrate antibodies (immunoglobulins) can be assigned to one of two distinct classes (called κ and λ) based on the amino acid sequence of their constant regions. Immunoglobulins can be divided into different classes based on the amino acid sequence of their heavy chain constant regions. There are mainly 5 classes of immunoglobulins: IgA, IgD, IgE, IgG, and IgM, some of which can be further divided into subclasses (isotypes), such as IgG1, IgG2, IgG3, IgG4, IgA1, and IgA2. The heavy chain constant regions corresponding to different classes of immunoglobulins are called α, δ, ε, γ, and µ, respectively. The subunit structure and three-dimensional configuration of different classes of immunoglobulins are well known to those skilled in the art.

The present invention also provides other polypeptides, such as fusion proteins comprising human antibodies or fragments thereof. In addition to almost full-length polypeptides, the present invention also includes fragments of the antibodies of the present invention. Usually, the fragment has at least about 50 consecutive amino acids, preferably at least about 50 consecutive amino acids, more preferably at least about 80 consecutive amino acids, and most preferably at least about 100 consecutive amino acids of the antibody of the present invention.

In the present invention, the antibody of the present invention also includes its conservative variants, which refer to polypeptides formed by substituting at most 10, preferably at most 8, more preferably at most 5, and most preferably at most 3 amino acids with amino acids of similar or close properties compared to the amino acid sequence of the antibody of the present invention. These conservative variant polypeptides are preferably produced by amino acid substitution according to Table A.

**Table A**

| Original Residue | Representative Substitution(s) | Preferred Substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Chimeric Antigen Receptor (CAR)

As used herein, the terms "chimeric antigen receptor of the present invention" and "CAR of the present invention" may be used interchangeably and refer to the chimeric antigen receptor according to the third aspect of the present invention.

The chimeric antigen receptor (CAR) of the present invention comprises an extracellular domain, a transmembrane domain, and an intracellular domain. The extracellular domain includes a target-specific binding element (also called an antigen-binding domain). The intracellular domain includes a costimulatory signaling region and a ζ chain portion. The costimulatory signaling region refers to a portion comprising the intracellular domain of a costimulatory molecule. Costimulatory molecules are cell surface molecules required for the effective response of lymphocytes to antigens, other than antigen receptors or their ligands.

A linker may be incorporated between the extracellular domain and the transmembrane domain of the CAR, or between the cytoplasmic domain and the transmembrane domain of the CAR. As used herein, the term "linker" generally refers to any oligopeptide or polypeptide that functions to connect the transmembrane domain to the extracellular or cytoplasmic domain of a polypeptide chain. The linker may include 0-300 amino acids, preferably 2 to 100 amino acids, and most preferably 3 to 50 amino acids.

In a preferred embodiment of the present invention, the extracellular domain of the CAR provided by the present invention comprises an antigen-binding domain targeting DLL3. When expressed in T cells, the CAR of the present invention can perform antigen recognition based on antigen-binding specificity. When it binds to its associated antigen, it affects tumor cells, leading to tumor cell growth arrest, induced death, or other effects, and results in the reduction or elimination of tumor burden in the patient. The antigen-binding domain is preferably fused to an intracellular domain from one or more of a costimulatory molecule and a ζ chain. Preferably, the antigen-binding domain is fused to an intracellular domain combining a 4-1BB signaling domain and a CD3ζ signaling domain.

As used herein, both "antigen-binding domain" and "single-chain antibody fragment" refer to a Fab fragment, Fab' fragment, F(ab')2 fragment, or single Fv fragment with antigen-binding activity. An Fv antibody contains a heavy chain variable region and a light chain variable region of an antibody but no constant region, and is the smallest antibody fragment with all antigen-binding sites. Generally, an Fv antibody also contains a polypeptide linker between the VH and VL domains and is capable of forming the structure required for antigen binding. The antigen-binding domain is usually an scFv (single-chain variable fragment). The size of an scFv is generally 1/6 of a complete antibody. A single-chain antibody is preferably an amino acid chain sequence encoded by a single nucleotide chain. As a preferred embodiment of the present invention, the scFv comprises an antibody that specifically recognizes the extracellular region of DLL3, preferably a single-chain antibody.

For the hinge region and transmembrane region (transmembrane domain), the CAR can be designed to include a transmembrane domain fused to the extracellular domain of the CAR. In one embodiment, a transmembrane domain naturally associated with one of the domains in the CAR is used. In some instances, the transmembrane domain may be selected or modified by amino acid substitution to avoid binding such a domain to the transmembrane domain of the same or different surface membrane proteins, thereby minimizing interactions with other members of the receptor complex.

Preferably, the structure of the CAR of the present invention includes a signal peptide, an antigen recognition sequence (antigen-binding domain), a linker region, a transmembrane region, a costimulatory factor signal region, and a CD3zeta signaling region (ζ chain portion), connected in the following order: L-scFv-H-TM-C-CD3ζ (I)

### NK Cell

Natural killer (NK) cells are a major type of immune effector cells that protect the body from viral infections and tumor cell invasion through non-antigen-specific pathways. In recent years, NK cells have shown great application prospects in adoptive cellular immunotherapy. NK cells have a wide range of sources, including peripheral blood, umbilical cord blood, embryonic stem cells (ESCs), induced pluripotent stem cells (iPSCs), etc.

NK-92 cells are an interleukin-2 (IL-2)-dependent NK cell line derived from peripheral blood mononuclear cells of a 50-year-old Caucasian male patient with acute non-Hodgkin's lymphoma. NK-92 cells are currently the only NK cell line approved by the FDA for clinical trials. This cell line has strong cytotoxicity, is economical, off-the-shelf, and easy to scale up production, has a short survival time after killing tumor cells, and is easy to expand *in vitro.* Most patients receiving the treatment of NK-92 cells do not exhibit rejection, and there is no risk of graft-versus-host disease. It does not express KIRs, is in a constitutively activated state, and has shown good clinical safety so far.

As used herein, the terms "CAR-NK cell", "CAR-NK", "CARNK", and "CAR-NK cell of the present invention" all refer to CAR-NK cells expressing the chimeric antigen receptor (CAR) of the first aspect of the present invention.

### Vector

Nucleic acid sequences encoding desired molecules can be obtained using recombinant methods known in the art, such as, for example, by screening a library from cells expressing the gene, by obtaining the gene from a vector known to include the gene, or by directly isolating it from cells and tissues containing the gene using standard techniques. Alternatively, the gene of interest can be produced synthetically.

The present invention also provides vectors into which the expression cassette of the present invention is inserted. Vectors derived from retroviruses such as lentiviruses are suitable tools for achieving long-term gene transfer because they allow long-term, stable integration of the transgene and its proliferation in daughter cells. Lentiviral vectors have advantages over vectors derived from oncogenic retroviruses such as murine leukemia virus in that they can transduce non-proliferating cells, such as hepatocytes. They also have the advantage of low immunogenicity.

Briefly, the expression cassette or nucleic acid sequence of the present invention is usually operably linked to a promoter and incorporated into an expression vector. The vector is suitable for replication and integration into eukaryotic cells. Typical cloning vectors contain transcriptional and translational terminators, initiation sequences, and promoters that can be used to regulate the expression of the desired nucleic acid sequence.

The expression constructs of the present invention can also be used for nucleic acid immunization and gene therapy using standard gene delivery protocols. Methods for gene delivery are known in the art. See, for example, U.S. Patent Nos. 5,399,346, 5,580,859, 5,589,466, incorporated herein by reference in their entirety. In another embodiment, the present invention provides gene therapy vectors.

The nucleic acid can be cloned into many types of vectors. For example, the nucleic acid can be cloned into vectors including, but not limited to, plasmids, phagemids, phage derivatives, animal viruses, and cosmids. Specific vectors of interest include expression vectors, replication vectors, probe generation vectors, and sequencing vectors.

Furthermore, the expression vector can be provided to the cell in the form of a viral vector. Viral vector technology is well known in the art and is described, for example, in Sambrook et al. (2001, Molecular Cloning: A Laboratory Manual, Cold Spring Harbor Laboratory, New York) and other virology and molecular biology manuals. Viruses that can be used as vectors include, but are not limited to, retroviruses, adenoviruses, adeno-associated viruses, herpesviruses, and lentiviruses. Generally, suitable vectors contain an origin of replication that functions in at least one organism, a promoter sequence, convenient restriction enzyme sites, and one or more selectable markers (e.g., those described in WO01/96584; WO01/29058; and U.S. Patent No. 6,326,193).

Many virus-based systems have been developed for transferring genes into mammalian cells. For example, retroviruses provide a convenient platform for gene delivery systems. The selected gene can be inserted into a vector and packaged into retroviral particles using techniques known in the art. The recombinant virus can then be isolated and delivered to target cells *in vivo* or *ex vivo.* Many retroviral systems are known in the art. In some embodiments, adenoviral vectors are used. Many adenoviral vectors are known in the art. In one embodiment, lentiviral vectors are used.

Additional promoter elements, such as enhancers, can regulate the frequency of transcription initiation. Usually, these are located in the region 30-110 bp upstream of the initiation site, although it has recently been shown that many promoters also contain functional elements downstream of the initiation site. The spacing between promoter elements is often flexible, so that promoter function is maintained when one element is inverted or moved relative to one another. In the thymidine kinase (tk) promoter, the spacing between promoter elements can be increased by 50 bp before activity begins to decrease. Depending on the promoter, individual elements can act cooperatively or independently to initiate transcription.

One example of a suitable promoter is the immediate early cytomegalovirus (CMV) promoter sequence. This promoter sequence is a strong constitutive promoter sequence capable of driving high-level expression of any polynucleotide sequence operably linked thereto. Another example of a suitable promoter is elongation factor-1α (EF-1α). However, other constitutive promoter sequences can also be used, including, but not limited to, the simian virus 40 (SV40) early promoter, mouse mammary tumor virus (MMTV), human immunodeficiency virus (HIV) long terminal repeat (LTR) promoter, MoMuLV promoter, avian leukemia virus promoter, Epstein-Barr virus immediate early promoter, Rous sarcoma virus promoter, and human gene promoters such as, but not limited to, the actin promoter, myosin promoter, heme promoter, and creatine kinase promoter. Furthermore, the present invention should not be limited to the use of constitutive promoters. Inducible promoters are also contemplated as part of the present invention. The use of inducible promoters provides a molecular switch that can turn on the expression of a polynucleotide sequence operably linked to the inducible promoter when such expression is desired, or turn it off when expression is not desired. Examples of inducible promoters include, but are not limited to, the metallothionein promoter, glucocorticoid promoter, progesterone promoter, and tetracycline promoter.

To assess the expression of the CAR polypeptide or portion thereof, the expression vector introduced into the cell may also contain either or both of a selectable marker gene or a reporter gene to facilitate the identification and selection of expressing cells from a population of cells sought to be transfected or infected by the viral vector. In other aspects, the selectable marker can be carried on a separate piece of DNA and used in a co-transfection procedure. Both the selectable marker and the reporter gene can be flanked by appropriate regulatory sequences to enable expression in the host cell. Useful selectable markers include, for example, antibiotic resistance genes such as neo, etc.

Reporter genes are used to identify potentially transfected cells and to evaluate the functionality of regulatory sequences. Generally, a reporter gene is a gene that is not present in or expressed by the recipient organism or tissue and that encodes a polypeptide whose expression is clearly indicated by some easily detectable property, such as enzymatic activity. Following the introduction of the DNA into the recipient cell, the expression of the reporter gene is assayed at an appropriate time. Suitable reporter genes may include genes encoding luciferase, β-galactosidase, chloramphenicol acetyltransferase, secreted alkaline phosphatase, or green fluorescent protein (e.g., those described in Ui-Tei et al., 2000 FEBS Letters 479:79-82). Suitable expression systems are well known and can be prepared using known techniques or obtained commercially. Generally, the construct with the minimal 5 flanking region showing the highest level of reporter gene expression is identified as the promoter. Such a promoter region can be linked to a reporter gene and used to evaluate the ability of an agent to modulate promoter-driven transcription.

Methods for introducing genes into cells and expressing genes into cells are known in the art. In the context of expression vectors, the vector can be easily introduced into a host cell, such as a mammalian, bacterial, yeast, or insect cell, by any method in the art. For example, the expression vector can be transferred into the host cell by physical, chemical, or biological means.

Physical methods for introducing polynucleotides into host cells include calcium phosphate precipitation, lipofection, particle bombardment, microinjection, electroporation, etc. Methods for producing cells containing vectors and/or exogenous nucleic acids are well known in the art. See, for example, Sambrook et al. (2001, Molecular Cloning: A Laboratory Manual, Cold Spring Harbor Laboratory, New York). A preferred method for introducing polynucleotides into host cells is calcium phosphate transfection.

Biological methods for introducing polynucleotides of interest into host cells include the use of DNA and RNA vectors. Viral vectors, particularly retroviral vectors, have become the most widely used method for inserting genes into mammalian, e.g., human, cells. Other viral vectors can be derived from lentiviruses, poxviruses, herpes simplex virus type I, adenoviruses, and adeno-associated viruses, etc. See, for example, U.S. Patent Nos. 5,350,674 and 5,585,362.

Chemical means for introducing polynucleotides into host cells include colloidal dispersion systems, such as macromolecular complexes, nanocapsules, microspheres, beads; and lipid-based systems, including oil-in-water emulsions, micelles, mixed micelles, and liposomes. An exemplary colloidal system for use as a delivery vehicle *in vitro* and *in vivo* is a liposome (e.g., an artificial membrane vesicle).

In the case of non-viral delivery systems, an exemplary delivery vehicle is a liposome. Lipid formulations are contemplated for use in introducing nucleic acids into host cells (*in vitro, ex vivo,* or *in vivo*). In another aspect, the nucleic acid may be associated with a lipid. Nucleic acid associated with a lipid may be encapsulated in the aqueous interior of a liposome, dispersed within the lipid bilayer of a liposome, attached to a liposome via a linking molecule associated with both the liposome and the oligonucleotide, entrapped in a liposome, complexed with a liposome, dispersed in a solution containing a lipid, mixed with a lipid, combined with a lipid, contained as a suspension in a lipid, contained in a micelle, or complexed with a micelle, or otherwise associated with a lipid. The lipid, lipid/DNA, or lipid/expression vector associated with the composition is not limited to any particular structure in solution. For example, they may exist in a bilayer structure, as micelles, or as "collapsed" structures. They may also simply be dispersed in solution, possibly forming aggregates of varying size or shape. Lipids are fatty substances that may be naturally occurring or synthetic lipids. For example, lipids include fatty droplets naturally occurring in the cytoplasm as well as such compounds that contain long-chain aliphatic hydrocarbons and their derivatives such as fatty acids, alcohols, amines, amino alcohols, and aldehydes.

In a preferred embodiment of the present invention, the vector is a retroviral vector.

### Therapeutic Applications

The present invention includes cells (e.g., NK cells, T cells, etc.) transduced with a retroviral vector (RV) encoding the antibody or CAR of the present invention. Transduced NK cells can induce a CAR-mediated NK-cell or T-cell response.

Thus, the present invention also provides a method for stimulating an NK cell-mediated immune response against a target cell population or tissue in a mammal, comprising the step of administering to the mammal NK cells expressing the CAR of the present invention.

In one embodiment, the present invention includes a type of cell therapy wherein NK cells are genetically modified to express the CAR of the present invention, and the CAR-NK cells are infused into a recipient in need thereof. The infused cells are capable of killing the recipient's tumor cells. Unlike antibody therapy, CAR-NK cells can persist *in vivo,* producing long-term persistence that can lead to sustained tumor control.

Treatable cancers include tumors that are not vascularized or are essentially not vascularized, as well as vascularized tumors. Cancers can include non-solid tumors (such as hematological tumors, e.g., leukemia and lymphoma) or can include solid tumors. Types of cancers to be treated with the CAR of the present invention include, but are not limited to, carcinomas, blastomas, and sarcomas, and certain leukemias or lymphoid malignancies, benign and malignant tumors, and malignancies such as sarcomas, carcinomas, and melanomas. Adult tumors/cancers and pediatric tumors/cancers are also included.

Solid tumors are abnormal masses of tissue that do not usually contain cysts or liquid areas. Solid tumors can be benign or malignant. Different types of solid tumors are named after the type of cell that forms them (such as sarcomas, carcinomas, and lymphomas). Examples of solid tumors such as sarcomas and carcinomas include fibrosarcoma, myxosarcoma, liposarcoma, mesothelioma, lymphoid malignancies, pancreatic cancer, and ovarian cancer.

The CAR-modified NK cells of the present invention can also be used as a type of vaccine for *ex vivo* immunization and/or *in vivo* therapy of mammals. Preferably, the mammal is a human.

### Pharmaceutical Composition

The antibody, fusion protein, or CAR-modified NK cells of the present invention can be administered alone or as a pharmaceutical composition in combination with diluents and/or other components such as IL-2, IL-15, IL-18, IL-21, or other cytokines or cell populations. Briefly, the pharmaceutical composition of the present invention may include a target cell population as described herein, in combination with one or more pharmaceutically or physiologically acceptable carriers, diluents, or excipients. Such compositions may include buffers such as neutral buffered saline, sulfate-buffered saline, etc.; carbohydrates such as glucose, mannose, sucrose, or dextran, mannitol; proteins; polypeptides or amino acids such as glycine; antioxidants; chelating agents such as EDTA or glutathione; adjuvants (e.g., aluminum hydroxide); and preservatives. The compositions of the present invention are preferably formulated for intravenous administration.

The pharmaceutical composition of the present invention can be administered in a manner suitable for the disease to be treated (or prevented). The quantity and frequency of administration will be determined by such factors as the condition of the patient, and the type and severity of the patient's disease-although an appropriate dosage can be determined by clinical trials.

When referring to an "immunologically effective amount", "anti-tumor effective amount", "tumor-inhibitory effective amount", or "therapeutic amount", the precise amount of the composition of the present invention to be administered can be determined by the physician, considering the age, weight, tumor size, degree of infection or metastasis, and individual differences in the condition of the patient (subject). It can generally be stated that a pharmaceutical composition comprising T cells as described herein can be administered at a dosage of 10⁴ to 10⁹ cells/kg body weight, preferably 10⁵ to 10⁶ cells/kg body weight (including all integer values within those ranges). The T cell composition can also be administered multiple times at these dosages. Cells can be administered by injection techniques known in immunotherapy (see, e.g., Rosenberg et al., New Eng. J. of Med. 319:1676, 1988). The optimal dosage and treatment regimen for a particular patient can be easily determined by those skilled in the medical field by monitoring the patient's signs of disease and thus adjusting the treatment.

### The Main Advantages of the Present Invention Include:

(1) The chimeric antigen receptor of the present invention has a specific anti-DLL3 scFv as its extracellular antigen-binding domain. The specific anti-DLL3 scFv is combined with a specific hinge region and an intracellular domain, and the formed CAR exhibits great killing ability against tumor cells, with low cytotoxicity and few side effects.
(2) The CAR-NK cells of the present invention have a high degree of activation and excellent cytotoxicity against DLL3-positive target cells.
(3) The antibody of the present invention has excellent binding ability and affinity kinetics to target cells, which is significantly superior to the commercially available positive control.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturers. Percentages and parts are calculated by weight unless otherwise stated.

### Example 1 Screening of Anti-DLL3 Antibodies

DLL3-related antibodies were obtained by immunizing humanized mice with antigen to generate antibody-producing region gene fragments, followed by hybridoma screening. Candidate scFvs were in the form of VL-(G4S)3-VH, and the sequences are shown in Table 1. The CDR sequences annotated using Kabat and Chothia are shown in Table 2 and Table 3. The following experiments were performed using antibodies in the form of scFv-human IgG Fc.

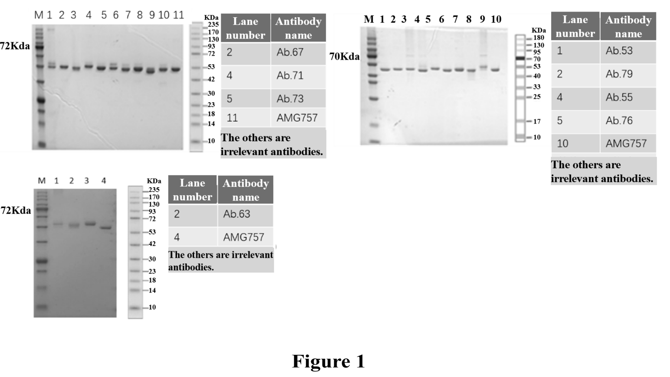

The results of SDS-PAGE detection of the expression and purification of anti-DLL3 scFv-Fc antibodies are shown in Figure 1. The results indicate that the antibody expression and purification are good. The anti-DLL3 scFv from Amgen's patent was selected as the positive control (AMG757).

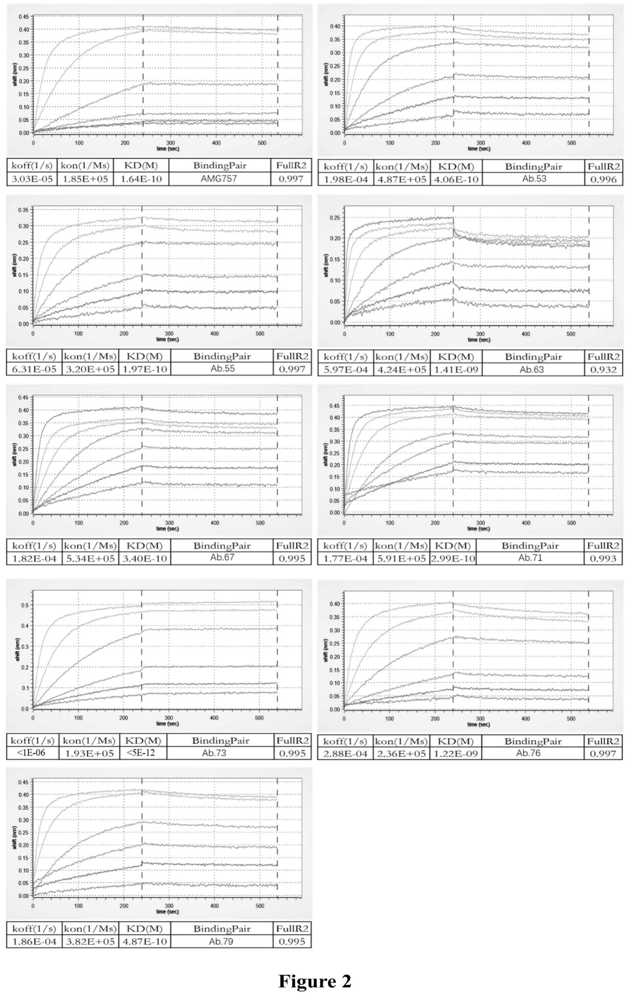

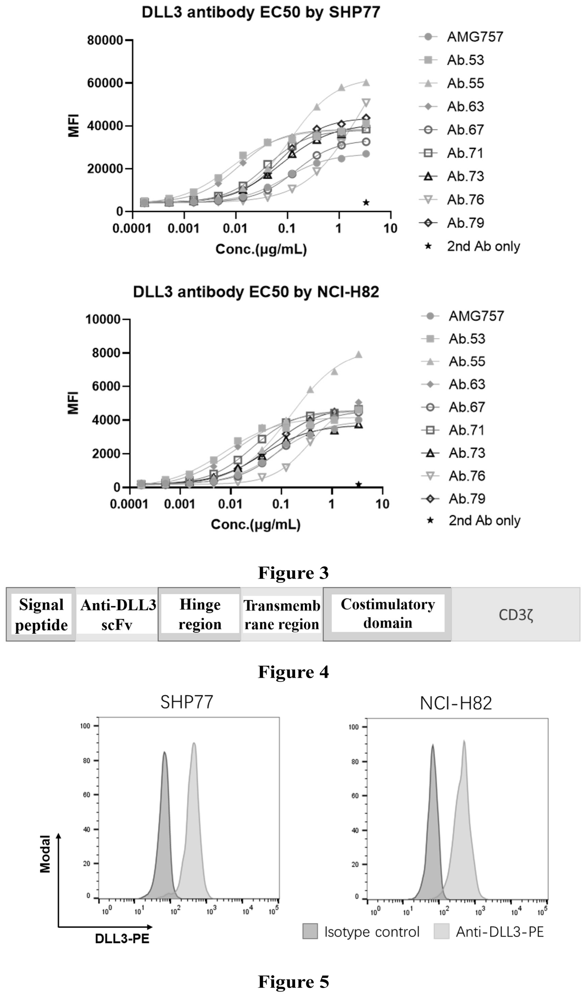

The Biolayer Interferometry (BLI) assay results of representative antibodies against human DLL3 protein are shown in Figure 2. All antibodies were subjected to flow cytometry binding analysis using two DLL3-positive cell lines, SHP77 and NCI-H82, and the results are shown in Figure 3. The BLI data and EC50 data of all antibodies are summarized in Table 4.

**Table 1 Anti-DLL3 Antibody scFv Sequences**

| Antibody Name | Amino Acid Sequence | SEQ ID NO: |
|---|---|---|
| Ab.76 | EIVMTQSPATLSVSPGERATLSCRASQSVSSNLAWYRQKPGQAPRLF IYGAYTRATDIPARFSGSGSGTEFTLTISSLQSEDFAVYYCQQYNNW PFTFGPGTKVDIKGGGGSGGGGSGGGGSEVQMVESGGVLVQPGMS LRLSCAASGFTFDDYAMHWVRQPPGKGLEWVSVISWNSGSIGYADS VKGRFTISRDNAKNSLYLQMNSLRAEDTAFYYCAKDLEGGVMDFD YWGQGTLVTVSS | 59 |
| Ab.55 | EVVMTQSPLSLPVTLGQPASVSCRSSQSLLYSDGNTYLIWFQQRPGQ SPRRLLYKLSTRDSGVPDRFSGSGSGTDFTLKISRVEAEDVGIYYCM QVTHWPYTFGQGTKLEIKGGGGSGGGGSGGGGSQVQLRESGPGLV KPSETLSLTCTVSGDSLKNSYWGWIRQPPGKGLEWIGYIYYSGTSSY NPSLESRVTFSADTSKNQFSLKLSSVTAADTAVYYCSREGSFDVWG RGTLVTVSS | 60 |
| Ab.67 | DVVVTQSPLSLPVTLGQPASISCRSSQSLVYSDGNTYLNWFQQRPGQ SPRRLVYQVSNRDSGVPDRFSGSGSGTDFTLKISGMEAEDAGVYYC MQGTHWPHTFGQGTKLEIKGGGGSGGGGSGGGGSEVHLVESGGGL VQPGGSLKLSCAASGFIFSGSSMHWVRQASGKGLEWIGRIRNKANN YATSYGASVQGRFTMSRDDSKNTAYLQMNSLKAEDTAVYYCTYLS GEFWGQGTLVTVSS | 61 |
| Ab.73 | DVVMTQSPLSLPVTLGQPASISCRSSQSLGYSDGDTYLIWFLQRPGQ SPRRLIYKVSNRDSGVPDRFSGSGSGTDFTLKISRVEAEDVGVYYCM QGTHWPYTFGQGTKLEIKGGGGSGGGGSGGGGSQVQVQESGPGLV KPSETLSLTCTVSTGSFSSHYWSWIRQPPGKSLEYIGYIYYTGTTNYN PSLKSRVTISVDTSKNQFSLNVNSVTAADTAVYYCAREGTFDIWGQ GTMVTVSS | 62 |
| Ab.71 | DIGMTQSPDSLAVSLGERATINCKSSQNVLPNSNKKHYLSWYQQKP GQPPKLLIYLASTRASGVPDRFSGSGSGTDFTLTISSLQAEDVAVYYC QQYYSSPPEWTFGQGTKVEIKGGGGSGGGGSGGGGSQVQLVESGG GVVQPGRSLRLSCVASGFTFSNCGMHWVRQAPGKGLEWVAIIWYD GNIKYYGDSVKGRFTISRDNSKNTLYLQMNSLRVEDTAVYYCGRHD YGGNSFDYWGQGTLVTVSS | 63 |
| Ab.53 | DIVMTQSPLSLSVTPGEPASISCRSSQSLLESNGYNYLDWYLQKPGQ SPQLLIYLGSNRASGVPDRFSGSGSGTDFTLKISRVEAEDVGIYYCM QALQTPLTFGGGTKVEIRGGGGSGGGGSGGGGSQVQLVQSGSELKK PGASVKVSCKASGYAFSNYAVNWVRQAPGQGLEWMGLINTYTENP TYAQDFTGRFVFSLDTSVTTAYLQITSLKTEDTAVYYCARGMGVFDI WGQGTMVTVSS | 64 |
| Ab.79 | DVVMTQSPLSLPVTLGQPASISCRSSQSLVYNDGNTYLTWFQQWPG QSPRRLIYKVSNRDSGVPDRFSGSGSGTDFTLKISRVEAEDIGVYHC MQGTHWPYTFGQGTKLEIKGGGGSGGGGSGGGGSQVQVQESGPGL VKSSETLSLTCTVSGGSFRSYYWSWIRQPPGKGLDYIGYMYYSGTT NYNPSLTNRVSISVDTSKNQFSLKLNSVTAADTAVYYCAREGTFDI WGQGTVVTVSS | 65 |
| Ab.63 | QIVLTQSPGTLSLSPGERATLSCRASQSVTRGYLAWYQQKPGQAPRL LFYGASNRATDIPDRFSGSGSGTDFTLIISRLEPEDFAVYYCQQYGISP LTFGGGTKVEIKGGGGSGGGGSGGGGSQVQLHESGPGLVKPSETLS LTCTVSGASISSYYWSWIRQPPGKGLEWIGYIYYSGTTNFNPSLKSR VTISVDTSKNQFSLRLSSVTTADTAVYYCANLALTGFHFDYWGQGT LVTVSS | 66 |
| AMG757 | EIVLTQSPGTLSLSPGERVTLSCRASQRVNNNYLAWYQQRPGQAPR LLIYGASSRATGIPDRFSGSGSGTDFTLTISRLEPEDFAVYYCQQYDR SPLTFGCGTKLEIKGGGGSGGGGSGGGGSQVQLQESGPGLVKPSETL SLTCTVSGGSISSYYWSWIRQPPGKCLEWIGYVYYSGTTNYNPSLKS RVTISVDTSKNQFSLKLSSVTAADTAVYYCASIAVTGFYFDYWGQG TLVTVSS | 67 |

**Table 2 CDR Sequences Based on Kabat**

| **Ab Name** | **VH CDR1** | **SE Q** | **VH CDR2** | **SE Q** | **VH CDR3** | **SE Q** | **VL CDR1** | **SE Q** | **VL CDR2** | **SE Q** | **VL CDR3** | **SE Q** |
|---|---|---|---|---|---|---|---|---|---|---|---|---|
| Ab.53 | NYA VN | 1 | LINTYTENPTYA QDFTG | 2 | GMGVFDI | 3 | RSSQSLLESNG YNYLD | 4 | LGSN RAS | 5 | MQALQT PLT | 6 |
| Ab.55 | NSY WG | 7 | YIYYSGTSSYNP SLES | 8 | EGSFDV | 9 | RSSQSLLYSDG NTYLI | 10 | KLST RDS | 11 | MQVTHW PYT | 12 |
| Ab.63 | SYY WS | 13 | YIYYSGTTNFNP SLKS | 14 | LALTGFH FDY | 15 | RASQSVTRGY LA | 16 | GASN RAT | 17 | QQYGISP LT | 18 |
| Ab.67 | GSSM H | 19 | RIRNKANNYATS YGASVQG | 20 | LSGEF | 21 | RSSQSLVYSD GNTYLN | 22 | QVSN RDS | 23 | MQGTHW PHT | 24 |
| Ab.71 | NCG MH | 25 | IIWYDGNIKYYG DSVKG | 26 | HDYGGN SFDY | 27 | KSSQNVLPNS NKKHYLS | 28 | LAST RAS | 29 | QQYYSSP PEWT | 30 |
| Ab.73 | SHY WS | 31 | YIYYTGTTNYNP SLKS | 32 | EGTFDI | 33 | RSSQSLGYSD GDTYLI | 34 | KVSN RDS | 35 | MQGTHW PYT | 36 |
| Ab.76 | DYA MH | 37 | VISWNSGSIGYA DSVKG | 38 | DLEGGV MDFDY | 39 | RASQSVSSNL A | 40 | GAYT RAT | 41 | QQYNNW PFT | 42 |
| Ab.79 | SYY WS | 13 | YMYYSGTTNYN PSLTN | 43 | EGTFDI | 33 | RSSQSLVYND GNTYLT | 44 | KVSN RDS | 35 | MQGTHW PYT | 36 |

| | | | | | | | | | | | | |
|---|---|---|---|---|---|---|---|---|---|---|---|---|
| SEQ: SEQ ID NO. | | | | | | | | | | | | |

**Table 3 CDR Sequences Based on Chothia**

| **Ab Name** | **VH CDR1** | **SE Q** | **VH CDR2** | **SE Q** | **VH CDR3** | **SE Q** | **VL CDR1** | **SE Q** | **VL CDR2** | **SE Q** | **VL CDR3** | **SE Q** |
|---|---|---|---|---|---|---|---|---|---|---|---|---|
| Ab.53 | GYAF SNY | 45 | NTYTE N | 46 | GMGVFDI | 3 | RSSQSLLESNG YNYLD | 4 | LGSN RAS | 5 | MQALQT PLT | 6 |
| Ab.55 | GDSL KNS | 47 | YYSGT | 48 | EGSFDV | 9 | RSSQSLLYSDG NTYLI | 10 | KLST RDS | 11 | MQVTHW PYT | 12 |
| Ab.63 | GASIS SY | 49 | YYSGT | 48 | LALTGFH FDY | 15 | RASQSVTRGYL A | 16 | GASN RAT | 17 | QQYGISP LT | 18 |
| Ab.67 | GFIFS GS | 50 | RNKAN NYA | 51 | LSGEF | 21 | RSSQSLVYSDG NTYLN | 22 | QVSN RDS | 23 | MQGTHW PHT | 24 |
| Ab.71 | GFTFS NC | 52 | WYDG NI | 53 | HDYGGNS FDY | 27 | KSSQNVLPNSN KKHYLS | 28 | LAST RAS | 29 | QQYYSSP PEWT | 30 |
| Ab.73 | TGSFS SH | 54 | YYTGT | 55 | EGTFDI | 33 | RSSQSLGYSDG DTYLI | 34 | KVSN RDS | 35 | MQGTHW PYT | 36 |
| Ab.76 | GFTF DDY | 56 | SWNSG S | 57 | DLEGGVM DFDY | 39 | RASQSVSSNLA | 40 | GAYT RAT | 41 | QQYNNW PFT | 42 |
| Ab.79 | GGSF RSY | 58 | YYSGT | 48 | EGTFDI | 33 | RSSQSLVYNDG NTYLT | 44 | KVSN RDS | 35 | MQGTHW PYT | 36 |

| | | | | | | | | | | | | |
|---|---|---|---|---|---|---|---|---|---|---|---|---|
| SEQ: SEQ ID NO. | | | | | | | | | | | | |

**Table 4 Summary of Anti-DLL3 Antibody Kinetic Data and EC50 Results (SCFV-FC)**

| Ab Name | KD (M) | ka (1/Ms) | kd (1/s) | FACS EC50(ng/mL, with NCI-H82) | FACS EC50(ng/mL, with SHP77) |
|---|---|---|---|---|---|
| AMG757 | 1.64E-10 | 1.85E+05 | 3.03E-05 | 82 | 89 |
| Ab.53 | 4.06E-10 | 4.87E+05 | 1.98E-04 | 7.2 | 8.1 |
| Ab.55 | 1.97E-10 | 3.20E+05 | 6.31E-05 | 150 | 110 |
| Ab.63 | 1.41E-09 | 4.24E+05 | 5.97E-04 | 12 | 11 |
| Ab.67 | 3.40E-10 | 5.34E+05 | 1.82E-04 | 110 | 140 |
| Ab.71 | 2.99E-10 | 5.91E+05 | 1.77E-04 | 27 | 39 |
| Ab.73 | <5E-12 | 1.93E+05 | <1E-06* | 42 | 77 |
| Ab.76 | 1.22E-09 | 2.36E+05 | 2.88E-04 | 380 | 2600 |
| Ab.79 | 4.87E-10 | 3.82E+05 | 1.86E-04 | 64 | 73 |

| | | | | | |
|---|---|---|---|---|---|
| *For Ab.73, since its koff is below the instrument detection limit (1E-06/s), it is represented as less than the lower limit in the table. | | | | | |

### Example 2 Design of Anti-DLL3 CAR Structure

The inventors found that an anti-DLL3 antibody with high affinity for DLL3 does not necessarily result in a CAR molecule with high killing performance. Therefore, the inventors conducted extensive screening. On the basis of the obtained antibodies, a large number of CAR molecule designs and screenings were carried out, and finally, molecules with excellent killing performance were obtained at the CAR level. Among them, the CARs corresponding to Ab.55, Ab.67, and Ab.76 showed exceptionally excellent performance in multi-round killing. The specific design is as follows:
The scFv of the anti-DLL3 CAR structure is derived from some of the antibodies screened in Example 1, and is named according to the antibody name and the order of the light chain and heavy chain in the scFv. Among them,
The structure of the anti-DLL3 CAR is shown in Figure 4, comprising a signal peptide (SP), scFv, hinge region, transmembrane domain (TM), costimulatory domain (CD), and CD3ζ domain. The signal peptides include the following sequences (Table 5), and the data of the present invention are based on CD8α SP, CD28 hinge, CD28 TM, and CD28 CD structure. The scFv is composed of VL-Linker-VH or VH-Linker-VL, where the linker can be (G4S)3 or (G4S)4, etc.

**Table 5 Signal Peptide Sequences**

| | | |
|---|---|---|
| IL-21SP | MRSSPGNMERIVICLMVIFLGTLV | SEQ ID No:68 |
| CD8αSP | MALPVTALLLPLALLLHAARP | SEQ ID No: 69 |
| CSF2SP | MWLQSLLLLGTVACSIS | SEQ ID No: 70 |
| DAP12SP | MGGLEPCSRLLLLPLLLAVSG | SEQ ID No: 71 |
| CD16SP | MWQLLLPTALLLLVSA | SEQ ID No: 72 |
| CD56SP | MLQTKDLIWTLFFLGTAVS | SEQ ID No: 73 |
| IL-8SP | MTSKLAVALLAAFLISAALC | SEQ ID No: 74 |

### Example 3 Detection of DLL3 Expression on Tumor Cell Surface

Experimental method: Flow cytometry was used to detect the expression of DLL3 on the surface of SHP77 and NCI-H82 tumor cells, and the detection method was as follows.

Take 2E5-3E5 target tumor cells to be detected, centrifuge at 300g for 5min at 4°C, and discard the supernatant. Add 200µL FACS Buffer (1% FBS dissolved in PBS), centrifuge at 300g for 5min at 4°C, and discard the supernatant.

Prepare the primary antibody solution at a concentration of 1µg/mL. Add 100µL/well of the primary antibody solution, mix by pipetting, and incubate at 4°C for 30min. After incubation, add 100µL FACS Buffer, centrifuge at 300g for 5min at 4°C, and discard the supernatant.

Add 200µL/well FACS Buffer, mix by pipetting, centrifuge at 300g for 5min at 4°C, and discard the supernatant. Repeat the above step once. Add 100µL/well of the secondary antibody solution, mix by pipetting, and incubate at 4°C for 30min. After incubation, add 100µL FACS Buffer, centrifuge at 300g for 5min at 4°C, and discard the supernatant. Add 200µL/well FACS Buffer, mix by pipetting, centrifuge at 300g for 5min at 4°C, and discard the supernatant. Repeat the above step once. Add 200µL/well FACS Buffer to resuspend, and detect using a flow cytometer.

Results: SHP77 and NCI-H82 cells express DLL3 protein on their surfaces (Figure 5).

### Example 4 Virus Packaging and NK92 Cell Infection

Experimental method: The retroviral packaging vectors are BaEV-TR and pCMV-gag-pol, respectively, and the vector carrying the CAR molecule is pMSCV. These vectors were designed by our laboratory and then sent to Genewiz for synthesis and extraction. The virus packaging, cell infection, and sorting processes were as follows:
Digest well-conditioned HEK-293T cells, resuspend in DMEM complete medium, and seed in 10cm culture dishes at 8E5/mL, 10mL/dish. Incubate in an incubator for 16h, then observe the cell density. When the density is about 90%, start plasmid transfection. Take centrifuge tubes, add 500µL Opti-MEM^{™} I Reduced Serum Medium (Gibco, 31985062) to each tube, respectively. In one centrifuge tube, add 7. 5µg BaEV-TR, 10µg pCMV-gag-pol, and 20µg of the corresponding pMSCV vector expressing CAR, mix well to obtain Opti-MEM-plasmid mixture; in another centrifuge tube, add 40µL PEIpro solution (polyplus, 115-010), mix well to obtain Opti-MEM-PEI mixture.

Add the Opti-MEM-plasmid mixture to the Opti-MEM-PEI mixture, pipette thoroughly to mix, and let stand at room temperature for 15min to form a transfection complex. After culture, collect the cell culture supernatant. Concentrate the virus using Lenti-X Concentrator (Takara, 631232).

Resuspend the virus with 100µL NK92 cell medium, take 5µL for titer determination, and store the remaining virus at 4°C temporarily. Titer determination was performed using K562 cells. During infection, 5µg/mL polybrene (Sigma-Aldrich, TR-1003) was added. 48h after infection, the positive rate of K562 was determined, and the virus titer was calculated.

Take 4E5 NK92 cells in a 6-well plate, add the virus concentrate at MOI=2, and add polybrene (Sigma-Aldrich, TR-1003) to a final concentration of 5µg/ml, mix well. Centrifuge the cells at 32°C and 800g for 1h, and incubate overnight. Take out the NK92, centrifuge and resuspend in fresh NK92 medium, and continue culturing for 4 days before flow cytometry detection.

Thoroughly mix the DLL3-CAR NK92 cells in culture by pipetting and count. According to the counting result, take 1E7 cells to be sorted into a centrifuge tube, centrifuge at 300g for 5min, and discard the supernatant. Resuspend the cells with 10mL MACS Buffer, centrifuge at 300g for 5min, and discard the supernatant. Resuspend the cells with 2mL of 3µg/mL Biotinylated Human DLL3 His, Avitag^{™} (Acro Biosystem, DL3-H82E4) dilution (diluted in PBS), and incubate at 4°C for 30min.

After incubation, wash and resuspend the cells, add 20 µL Anti-Biotin MicroBeads (Miltenyi, 130-090-485), and mix thoroughly. Incubate at 4°C for 15min. After incubation, wash and resuspend the cells. Pass through an LS adsorption column (Miltenyi, 130-042-401) loaded on a magnetic stand to elute the cells labeled with magnetic beads. Take 200µL of the sorted cells for NC-200 counting.

Resuspend the sorted cells and incubate in a 37°C, 5% CO₂ incubator. Determine the CAR positive rate 4 days after sorting. Take 1E5-2E5 cells to be tested, centrifuge at 700g for 2min at 4°C, and discard the supernatant. Wash, centrifuge, and discard the supernatant.

After mixing, add 100 µL of antigen protein suspension to each sample at a concentration of 2µg/mL, mix by pipetting, and incubate at 4°C for 30min-60min. After incubation, wash, centrifuge, and discard the supernatant.

Prepare the detection antibody streptavidin-PE (Biolegend, #405203) at a dilution ratio of 1:200. After mixing, add 100 µL of the detection antibody to each sample, mix by pipetting, and incubate at 4°C for 30min-60min.

After incubation, wash, centrifuge, and discard the supernatant. Add 200 µL/sample FACS Buffer to resuspend, and detect with a flow cytometer.

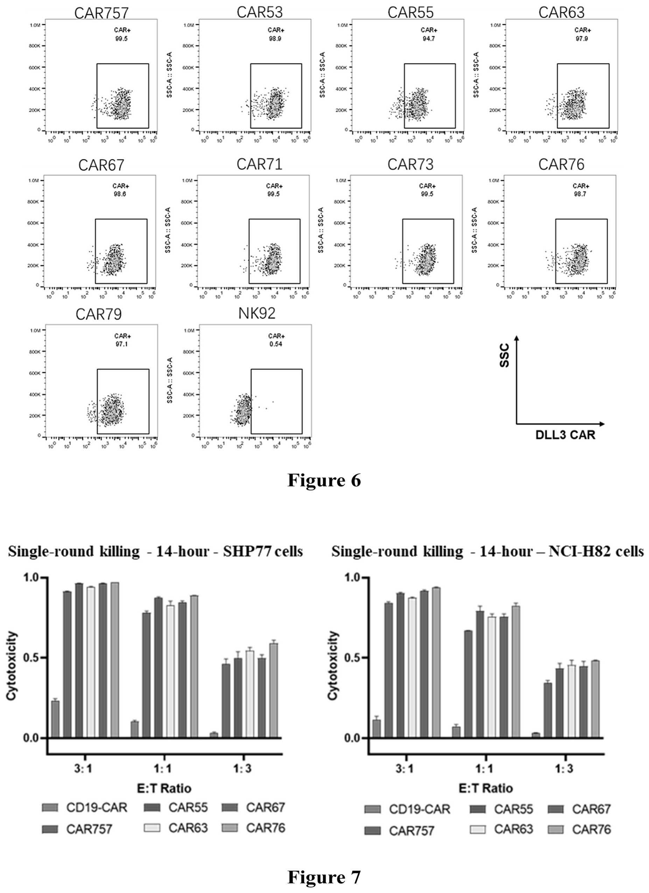

Experimental results: The CAR detection results of the sorted CAR-NK92 cell lines are shown in Figure 6.

Results indicated that after NK92 cells were infected with the virus and sorted by magnetic beads, the positive rate of anti-DLL3 CAR in each group of NK92 cells reached more than 95% except that the positive rate of CAR55 was 94.7%.

### Example 5 Detection of Killing Ability of Anti-DLL3 CAR-NK92 Against Target Cells

Experimental method: The killing ability of anti-DLL3 CAR-NK92 cells was detected using different methods.

### 5.1 Single-Round Killing

The Luciferase reporter gene was used to detect the single-round killing ability of anti-DLL3 CAR-NK92 against SHP77-Luciferase and NCI-H82-Luciferase target cells, with anti-CD19 CD19-CAR as the negative control. The specific operation steps were as follows:
Withdraw IL-2 from the culture medium of the CAR-NK92 effector cells for the killing experiment 24h in advance.

Digest the target cells, neutralize, mix thoroughly by pipetting and count, then resuspend with an appropriate volume of RPMI1640 complete medium. Transfer the target cells to a 96-well flat-bottom plate at 2E4/well, 100µL/well. Mix the effector cells thoroughly by pipetting and count, take an appropriate number of effector cells, centrifuge at 300g for 5min at room temperature, and resuspend with an appropriate volume of RPMI1640 complete medium. Transfer the effector cells to the 96-well flat-bottom plate of target cells at 50µL/well, and co-culture in a 37°C, 5% CO₂ incubator for an appropriate time. Add 60µL ONE-Glo to each well and mix. After reacting for 3min, mix thoroughly, take 130µL of the reaction solution into a 96-well white flat-bottom plate, detect the luminescence signal intensity using a microplate reader, and then calculate the cytotoxicity.

The killing results recorded by the Luciferase reporter gene method are shown in Figure 7. It can be found that under the experimental conditions, CAR55, CAR67, and CAR76 have good killing effects on both target cells.

### 5.2 Multi-Round Killing

The cell imaging multi-mode detection system (Cytation C7) was used to detect the multi-round killing ability of anti-DLL3 CAR-NK92 against SHP77-GFP and NCI-H82-GFP target cells, with anti-CD19 CD19-CAR as the negative control. The specific operation steps were as follows:
Withdraw IL-2 from the culture medium of the NK92 cells 24h in advance.

Take a 96-well plate for imaging and coat with poly-D-lysine for later use. Digest and disperse the target cells thoroughly, and seed them in the well plate at 2E4/well, 100µL/well. After seeding, cells attach at 37°C for 6 hours. Take photos of the well plate using Cytation C7 as the baseline. Slowly add 50µL/well of effector cells according to the experimentally set effector-to-target ratio (E: T), place the well plate into the Cytation C7 instrument, and start taking photos and recording after 30min.

When the first round of killing is about to reach the target cell detection baseline or the target cell reading is about to stop decreasing, start coating the 96-well plate for the second round of killing and seeding the target cells at 2E4/well, 100µL/well.

After the first round of killing reaches the target cell detection baseline or the target cell reading stops decreasing, thoroughly mix the solution in the wells of the first round of killing by pipetting, transfer them to the coated 96-well plate for the second round of killing, and start taking photos and recording after 30min.

### Experimental results:

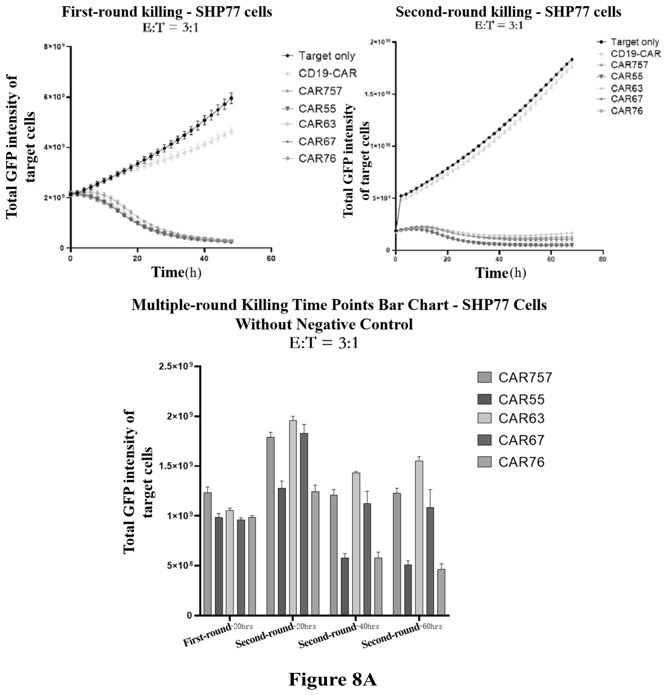

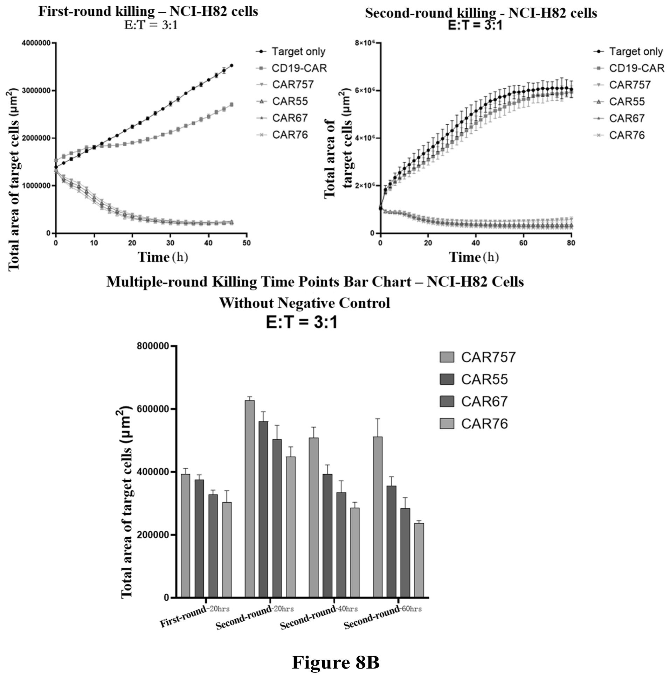

The single-round killing results of SHP77 and NCI-H82 cells by anti-DLL3 CAR-NK92 are shown in Figure 7, and the multi-round killing results are shown in Figures 8A and 8B. Under the experimental conditions, each group of CAR-NK92 has a good killing effect on target cells.

Results indicated that at a ratio of E: T=1: 1, in the first round of killing (14 hours), compared with the positive control CAR757, CAR76 can exhibit a specific killing activity greater than 82%.

Based on the experiments of the present invention, CAR76, CAR55, and CAR67 have excellent specific killing activity.

### Discussion

In single-round and multi-round killing, CAR76 has better specific killing activity than the positive control CAR757. However, surprisingly, the affinity (KD value) of the scFv antibody Ab76 corresponding to CAR76 is lower than that of the positive control.

In addition, compared with the positive control, Ab63 has an excellent EC50 value for NCI-H82, indicating its excellent killing activity. In the cell single-round killing activity experiment, it has good short-term CAR function killing activity (Figure 7), but is inferior to the positive control in CAR function multi-round killing (Figures 8A and 8B).

Based on the complex biological environment of the interaction between NK cells and target cells, the target affinity (KD value) of the antibody and the EC50 data for target cells are for reference only, and cannot obviously indicate that CAR-NK cells constructed using high-affinity antibodies as elements will necessarily have potent tumor cell killing performance.

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

## Claims

1. A DLL3-targeting antibody or an antigen-binding fragment thereof, wherein the antibody or antigen-binding fragment thereof comprises a heavy chain variable region and a light chain variable region, and wherein the heavy chain variable region and light chain variable region comprise complementarity determining regions (CDRs) selected from the group consisting of:
(1) the heavy chain variable region comprises the following complementarity determining regions (CDRs):
HCDR1 set forth in SEQ ID NO: 37,
HCDR2 set forth in SEQ ID NO: 38, and
HCDR3 set forth in SEQ ID NO: 39;
and the light chain variable region comprises the following complementarity determining regions (CDRs):
LCDR1 set forth in SEQ ID NO: 40,
LCDR2 set forth in SEQ ID NO: 41, and
LCDR3 set forth in SEQ ID NO: 42; or
(2) the heavy chain variable region comprises the following complementarity determining regions (CDRs):
HCDR1 set forth in SEQ ID NO: 7,
HCDR2 set forth in SEQ ID NO: 8, and
HCDR3 set forth in SEQ ID NO: 9;
and the light chain variable region comprises the following complementarity determining regions (CDRs):
LCDR1 set forth in SEQ ID NO: 10,
LCDR2 set forth in SEQ ID NO: 11, and
LCDR3 set forth in SEQ ID NO: 12; or
(3) the heavy chain variable region comprises the following complementarity determining regions (CDRs):
HCDR1 set forth in SEQ ID NO: 19,
HCDR2 set forth in SEQ ID NO: 20, and
HCDR3 set forth in SEQ ID NO: 21;
and the light chain variable region comprises the following complementarity determining regions (CDRs):
LCDR1 set forth in SEQ ID NO: 22,
LCDR2 set forth in SEQ ID NO: 23, and
LCDR3 set forth in SEQ ID NO: 24; or
(4) the heavy chain variable region comprises the following complementarity determining regions (CDRs):
HCDR1 set forth in SEQ ID NO: 31,
HCDR2 set forth in SEQ ID NO: 32, and
HCDR3 set forth in SEQ ID NO: 33;
and the light chain variable region comprises the following complementarity determining regions (CDRs):
LCDR1 set forth in SEQ ID NO: 34,
LCDR2 set forth in SEQ ID NO: 35, and
LCDR3 set forth in SEQ ID NO: 36; or
(5) the heavy chain variable region comprises the following complementarity determining regions (CDRs):
HCDR1 set forth in SEQ ID NO: 25,
HCDR2 set forth in SEQ ID NO: 26, and
HCDR3 set forth in SEQ ID NO: 27;
and the light chain variable region comprises the following complementarity determining regions (CDRs):
LCDR1 set forth in SEQ ID NO: 28,
LCDR2 set forth in SEQ ID NO: 29, and
LCDR3 set forth in SEQ ID NO: 30; or
(6) the heavy chain variable region comprises the following complementarity determining regions (CDRs):
HCDR1 set forth in SEQ ID NO: 1,
HCDR2 set forth in SEQ ID NO: 2, and
HCDR3 set forth in SEQ ID NO: 3;
and the light chain variable region comprises the following complementarity determining regions (CDRs):
LCDR1 set forth in SEQ ID NO: 4,
LCDR2 set forth in SEQ ID NO: 5, and
LCDR3 set forth in SEQ ID NO: 6; or
(7) the heavy chain variable region comprises the following complementarity determining regions (CDRs):
HCDR1 set forth in SEQ ID NO: 13,
HCDR2 set forth in SEQ ID NO: 43, and
HCDR3 set forth in SEQ ID NO: 33;
and the light chain variable region comprises the following complementarity determining regions (CDRs):
LCDR1 set forth in SEQ ID NO: 44,
LCDR2 set forth in SEQ ID NO: 35, and
LCDR3 set forth in SEQ ID NO: 36; or
(8) the heavy chain variable region comprises the following complementarity determining regions (CDRs):
HCDR1 set forth in SEQ ID NO: 13,
HCDR2 set forth in SEQ ID NO: 14, and
HCDR3 set forth in SEQ ID NO: 15;
and the light chain variable region comprises the following complementarity determining regions (CDRs):
LCDR1 set forth in SEQ ID NO: 16,
LCDR2 set forth in SEQ ID NO: 17, and
LCDR3 set forth in SEQ ID NO: 18;
wherein the CDR sequences are identified based on the Kabat numbering scheme; or
the heavy chain variable region and light chain variable region of the antibody or antigen-binding fragment thereof comprise complementarity determining regions (CDRs) selected from the group consisting of:
(1) the heavy chain variable region comprises the following complementarity determining regions (CDRs):
HCDR1 set forth in SEQ ID NO: 56,
HCDR2 set forth in SEQ ID NO: 57, and
HCDR3 set forth in SEQ ID NO: 39;
and the light chain variable region comprises the following complementarity determining regions (CDRs):
LCDR1 set forth in SEQ ID NO: 40,
LCDR2 set forth in SEQ ID NO: 41, and
LCDR3 set forth in SEQ ID NO: 42; or
(2) the heavy chain variable region comprises the following complementarity determining regions (CDRs):
HCDR1 set forth in SEQ ID NO: 47,
HCDR2 set forth in SEQ ID NO: 48, and
HCDR3 set forth in SEQ ID NO: 9;
and the light chain variable region comprises the following complementarity determining regions (CDRs):
LCDR1 set forth in SEQ ID NO: 10,
LCDR2 set forth in SEQ ID NO: 11, and
LCDR3 set forth in SEQ ID NO: 12; or
(3) the heavy chain variable region comprises the following complementarity determining regions (CDRs):
HCDR1 set forth in SEQ ID NO: 50,
HCDR2 set forth in SEQ ID NO: 51, and
HCDR3 set forth in SEQ ID NO: 21;
and the light chain variable region comprises the following complementarity determining regions (CDRs):
LCDR1 set forth in SEQ ID NO: 22,
LCDR2 set forth in SEQ ID NO: 23, and
LCDR3 set forth in SEQ ID NO: 24; or
(4) the heavy chain variable region comprises the following complementarity determining regions (CDRs):
HCDR1 set forth in SEQ ID NO: 54,
HCDR2 set forth in SEQ ID NO: 55, and
HCDR3 set forth in SEQ ID NO: 33;
and the light chain variable region comprises the following complementarity determining regions (CDRs):
LCDR1 set forth in SEQ ID NO: 34,
LCDR2 set forth in SEQ ID NO: 35, and
LCDR3 set forth in SEQ ID NO: 36; or
(5) the heavy chain variable region comprises the following complementarity determining regions (CDRs):
HCDR1 set forth in SEQ ID NO: 52,
HCDR2 set forth in SEQ ID NO: 53, and
HCDR3 set forth in SEQ ID NO: 27;
and the light chain variable region comprises the following complementarity determining regions (CDRs):
LCDR1 set forth in SEQ ID NO: 28,
LCDR2 set forth in SEQ ID NO: 29, and
LCDR3 set forth in SEQ ID NO: 30; or
(6) the heavy chain variable region comprises the following complementarity determining regions (CDRs):
HCDR1 set forth in SEQ ID NO: 45,
HCDR2 set forth in SEQ ID NO: 46, and
HCDR3 set forth in SEQ ID NO: 3;
and the light chain variable region comprises the following complementarity determining regions (CDRs):
LCDR1 set forth in SEQ ID NO: 4,
LCDR2 set forth in SEQ ID NO: 5, and
LCDR3 set forth in SEQ ID NO: 6; or
(7) the heavy chain variable region comprises the following complementarity determining regions (CDRs):
HCDR1 set forth in SEQ ID NO: 58,
HCDR2 set forth in SEQ ID NO: 48, and
HCDR3 set forth in SEQ ID NO: 33;
and the light chain variable region comprises the following complementarity determining regions (CDRs):
LCDR1 set forth in SEQ ID NO: 44,
LCDR2 set forth in SEQ ID NO: 35, and
LCDR3 set forth in SEQ ID NO: 36; or
(8) the heavy chain variable region comprises the following complementarity determining regions (CDRs):
HCDR1 set forth in SEQ ID NO: 49,
HCDR2 set forth in SEQ ID NO: 48, and
HCDR3 set forth in SEQ ID NO: 15;
and the light chain variable region comprises the following complementarity determining regions (CDRs):
LCDR1 set forth in SEQ ID NO: 16,
LCDR2 set forth in SEQ ID NO: 17, and
LCDR3 set forth in SEQ ID NO: 18;
wherein the CDR sequences are identified based on the Chothia numbering scheme.

2. A recombinant protein, comprising:
(i) the antibody or antigen-binding fragment thereof according to claim 1;
and (ii) an optional tag sequence facilitating expression and/or purification.

3. A chimeric antigen receptor (CAR), wherein the antigen-binding domain of the chimeric antigen receptor contains an antibody single-chain variable region sequence (scFv) targeting DLL3, and wherein the heavy chain variable region and light chain variable region of the scFv comprise complementarity determining regions (CDRs) selected from the group consisting of:
(1) the heavy chain variable region comprises the following complementarity determining regions (CDRs):
HCDR1 set forth in SEQ ID NO: 37,
HCDR2 set forth in SEQ ID NO: 38, and
HCDR3 set forth in SEQ ID NO: 39;
and the light chain variable region comprises the following complementarity determining regions (CDRs):
LCDR1 set forth in SEQ ID NO: 40,
LCDR2 set forth in SEQ ID NO: 41, and
LCDR3 set forth in SEQ ID NO: 42; or
(2) the heavy chain variable region comprises the following complementarity determining regions (CDRs):
HCDR1 set forth in SEQ ID NO: 7,
HCDR2 set forth in SEQ ID NO: 8, and
HCDR3 set forth in SEQ ID NO: 9;
and the light chain variable region comprises the following complementarity determining regions (CDRs):
LCDR1 set forth in SEQ ID NO: 10,
LCDR2 set forth in SEQ ID NO: 11, and
LCDR3 set forth in SEQ ID NO: 12; or
(3) the heavy chain variable region comprises the following complementarity determining regions (CDRs):
HCDR1 set forth in SEQ ID NO: 19,
HCDR2 set forth in SEQ ID NO: 20, and
HCDR3 set forth in SEQ ID NO: 21;
and the light chain variable region comprises the following complementarity determining regions (CDRs):
LCDR1 set forth in SEQ ID NO: 22,
LCDR2 set forth in SEQ ID NO: 23, and
LCDR3 set forth in SEQ ID NO: 24; or
(4) the heavy chain variable region comprises the following complementarity determining regions (CDRs):
HCDR1 set forth in SEQ ID NO: 31,
HCDR2 set forth in SEQ ID NO: 32, and
HCDR3 set forth in SEQ ID NO: 33;
and the light chain variable region comprises the following complementarity determining regions (CDRs):
LCDR1 set forth in SEQ ID NO: 34,
LCDR2 set forth in SEQ ID NO: 35, and
LCDR3 set forth in SEQ ID NO: 36; or
(5) the heavy chain variable region comprises the following complementarity determining regions (CDRs):
HCDR1 set forth in SEQ ID NO: 25,
HCDR2 set forth in SEQ ID NO: 26, and
HCDR3 set forth in SEQ ID NO: 27;
and the light chain variable region comprises the following complementarity determining regions (CDRs):
LCDR1 set forth in SEQ ID NO: 28,
LCDR2 set forth in SEQ ID NO: 29, and
LCDR3 set forth in SEQ ID NO: 30; or
(6) the heavy chain variable region comprises the following complementarity determining regions (CDRs):
HCDR1 set forth in SEQ ID NO: 1,
HCDR2 set forth in SEQ ID NO: 2, and
HCDR3 set forth in SEQ ID NO: 3;
and the light chain variable region comprises the following complementarity determining regions (CDRs):
LCDR1 set forth in SEQ ID NO: 4,
LCDR2 set forth in SEQ ID NO: 5, and
LCDR3 set forth in SEQ ID NO: 6; or
(7) the heavy chain variable region comprises the following complementarity determining regions (CDRs):
HCDR1 set forth in SEQ ID NO: 13,
HCDR2 set forth in SEQ ID NO: 43, and
HCDR3 set forth in SEQ ID NO: 33;
and the light chain variable region comprises the following complementarity determining regions (CDRs):
LCDR1 set forth in SEQ ID NO: 44,
LCDR2 set forth in SEQ ID NO: 35, and
LCDR3 set forth in SEQ ID NO: 36; or
(8) the heavy chain variable region comprises the following complementarity determining regions (CDRs):
HCDR1 set forth in SEQ ID NO: 13,
HCDR2 set forth in SEQ ID NO: 14, and
HCDR3 set forth in SEQ ID NO: 15;
and the light chain variable region comprises the following complementarity determining regions (CDRs):
LCDR1 set forth in SEQ ID NO: 16,
LCDR2 set forth in SEQ ID NO: 17, and
LCDR3 set forth in SEQ ID NO: 18;
wherein the CDR sequences are identified based on the Kabat numbering scheme; or
the heavy chain variable region and light chain variable region of the antibody or antigen-binding fragment thereof comprise complementarity determining regions (CDRs) selected from the group consisting of:
(1) the heavy chain variable region comprises the following complementarity determining regions (CDRs):
HCDR1 set forth in SEQ ID NO: 56,
HCDR2 set forth in SEQ ID NO: 57, and
HCDR3 set forth in SEQ ID NO: 39;
and the light chain variable region comprises the following complementarity determining regions (CDRs):
LCDR1 set forth in SEQ ID NO: 40,
LCDR2 set forth in SEQ ID NO: 41, and
LCDR3 set forth in SEQ ID NO: 42; or
(2) the heavy chain variable region comprises the following complementarity determining regions (CDRs):
HCDR1 set forth in SEQ ID NO: 47,
HCDR2 set forth in SEQ ID NO: 48, and
HCDR3 set forth in SEQ ID NO: 9;
and the light chain variable region comprises the following complementarity determining regions (CDRs):
LCDR1 set forth in SEQ ID NO: 10,
LCDR2 set forth in SEQ ID NO: 11, and
LCDR3 set forth in SEQ ID NO: 12; or
(3) the heavy chain variable region comprises the following complementarity determining regions (CDRs):
HCDR1 set forth in SEQ ID NO: 50,
HCDR2 set forth in SEQ ID NO: 51, and
HCDR3 set forth in SEQ ID NO: 21;
and the light chain variable region comprises the following complementarity determining regions (CDRs):
LCDR1 set forth in SEQ ID NO: 22,
LCDR2 set forth in SEQ ID NO: 23, and
LCDR3 set forth in SEQ ID NO: 24; or
(4) the heavy chain variable region comprises the following complementarity determining regions (CDRs):
HCDR1 set forth in SEQ ID NO: 54,
HCDR2 set forth in SEQ ID NO: 55, and
HCDR3 set forth in SEQ ID NO: 33;
and the light chain variable region comprises the following complementarity determining regions (CDRs):
LCDR1 set forth in SEQ ID NO: 34,
LCDR2 set forth in SEQ ID NO: 35, and
LCDR3 set forth in SEQ ID NO: 36; or
(5) the heavy chain variable region comprises the following complementarity determining regions (CDRs):
HCDR1 set forth in SEQ ID NO: 52,
HCDR2 set forth in SEQ ID NO: 53, and
HCDR3 set forth in SEQ ID NO: 27;
and the light chain variable region comprises the following complementarity determining regions (CDRs):
LCDR1 set forth in SEQ ID NO: 28,
LCDR2 set forth in SEQ ID NO: 29, and
LCDR3 set forth in SEQ ID NO: 30; or
(6) the heavy chain variable region comprises the following complementarity determining regions (CDRs):
HCDR1 set forth in SEQ ID NO: 45,
HCDR2 set forth in SEQ ID NO: 46, and
HCDR3 set forth in SEQ ID NO: 3;
and the light chain variable region comprises the following complementarity determining regions (CDRs):
LCDR1 set forth in SEQ ID NO: 4,
LCDR2 set forth in SEQ ID NO: 5, and
LCDR3 set forth in SEQ ID NO: 6; or
(7) the heavy chain variable region comprises the following complementarity determining regions (CDRs):
HCDR1 set forth in SEQ ID NO: 58,
HCDR2 set forth in SEQ ID NO: 48, and
HCDR3 set forth in SEQ ID NO: 33;
and the light chain variable region comprises the following complementarity determining regions (CDRs):
LCDR1 set forth in SEQ ID NO: 44,
LCDR2 set forth in SEQ ID NO: 35, and
LCDR3 set forth in SEQ ID NO: 36; or
(8) the heavy chain variable region comprises the following complementarity determining regions (CDRs):
HCDR1 set forth in SEQ ID NO: 49,
HCDR2 set forth in SEQ ID NO: 48, and
HCDR3 set forth in SEQ ID NO: 15;
and the light chain variable region comprises the following complementarity determining regions (CDRs):
LCDR1 set forth in SEQ ID NO: 16,
LCDR2 set forth in SEQ ID NO: 17, and
LCDR3 set forth in SEQ ID NO: 18;
wherein the CDR sequences are identified based on the Chothia numbering scheme.

4. A polynucleotide encoding the antibody or antigen-binding fragment thereof according to claim 1, the recombinant protein according to claim 2, or the chimeric antigen receptor (CAR) according to claim 3.

5. A vector comprising the polynucleotide according to claim 4.

6. A host cell, comprising the vector according to claim 5, or having an exogenous polynucleotide according to claim 4 integrated into its chromosome.

7. A method for preparing CAR-NK cells or CAR-T cells, wherein the CAR-NK cells or CAR-T cells express the chimeric antigen receptor according to claim 3, comprising the following steps:
transducing the polynucleotide according to claim 4 of the present invention or the vector according to claim 5 of the present invention into NK cells or T cells, thereby obtaining the CAR-NK cells or CAR-T cells.

8. A pharmaceutical composition comprising the antibody or antigen-binding fragment thereof according to claim 1, the recombinant protein according to claim 2, the chimeric antigen receptor according to claim 3, the polynucleotide according to claim 4, the vector according to claim 5, or the host cell according to claim 6, and a pharmaceutically acceptable carrier, diluent or excipient.

9. An immunoconjugate comprising:
(a) an antibody moiety selected from the group consisting of: the antibody or antigen-binding fragment thereof according to claim 1, the recombinant protein according to claim 2, and a combination thereof; and
(b) a conjugated moiety coupled to the antibody moiety, wherein the conjugated moiety is selected from the group consisting of: a detectable label, a drug, a toxin, a cytokine, a radionuclide, an enzyme, and a combination thereof.

10. Use of the antibody or antigen-binding fragment thereof according to claim 1, the recombinant protein according to claim 2, the chimeric antigen receptor according to claim 3, the polynucleotide according to claim 4, the vector according to claim 5, the host cell according to claim 6, the pharmaceutical composition according to claim 8, or the immunoconjugate according to claim 9, for:
(a) manufacturing a detection reagent or kit; and/or
(b) manufacturing a medicament or preparation for preventing and/or treating DLL3-associated diseases.

11. The use of claim 10, wherein the solid tumor is selected from the group consisting of: breast cancer, gastric cancer, lung cancer, ovarian cancer, colorectal cancer, pancreatic cancer, endometrial cancer, melanoma, mesothelioma, and a combination thereof.

12. A method for *in vitro* detection (including diagnostic or non-diagnostic) of DLL3 protein in a sample, comprising the steps of:
(1) contacting the sample with the antibody or antigen-binding fragment thereof according to claim 1, or the recombinant protein according to claim 2;
(2) detecting whether an antigen-antibody complex is formed, wherein the formation of the complex indicates the presence of DLL3 protein in the sample.

13. A method for preparing a recombinant polypeptide, comprising:
(a) culturing the host cell according to claim 6 under conditions suitable for expression;
(b) isolating the recombinant polypeptide from the culture, wherein the recombinant polypeptide is the antibody or antigen-binding fragment thereof according to claim 1, or the recombinant protein according to claim 2.

14. A detection plate comprising: a substrate (support plate) and a test strip, wherein the test strip contains the antibody or antigen-binding fragment thereof according to claim 1, the recombinant protein according to claim 2, the immunoconjugate according to claim 9, or a combination thereof.

15. A kit comprising:
(1) a first container containing the antibody or antigen-binding fragment thereof according to claim 1; and/or
(2) a second container containing a secondary antibody against the antibody according to claim 1;
or, the kit comprises the detection plate according to claim 14.
